(19) **Europäisches Patentamt / European Patent Office / Office européen des brevets**

(11) **EP 4 253 560 A1**

(12) # EUROPEAN PATENT APPLICATION

(43) Date of publication:
04.10.2023  Bulletin 2023/40

(21) Application number: 22382308.9

(22) Date of filing: 31.03.2022

(51) International Patent Classification (IPC):
*C12Q 1/6818* (2018.01)    *C12Q 1/6844* (2018.01)

(52) Cooperative Patent Classification (CPC):
(C-Sets available)
**C12Q 1/6818; C12Q 1/6844**              (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Consejo Superior de Investigaciones Cientificas**
**28006 Madrid (ES)**

(72) Inventors:
• **FERNANDEZ FRAGA, Mario**
**33940 EL ENTREGO (Asturias) (ES)**
• **FERNANDEZ FERNANDEZ, AGUSTIN**
**33940 EL ENTREGO (Asturias) (ES)**
• **TEJEDOR VAQUERO, JUAN RAMON**
**33940 EL ENTREGO (Asturias) (ES)**

(74) Representative: **Pons**
**Glorieta Rubén Darío 4**
**28010 Madrid (ES)**

Remarks:
The complete document including Reference Table(s) and the Sequence Listing(s) can be downloaded from the EPO website

(54) **ENHANCED DETECTION OF SINGLE-STRANDED NUCLEIC ACIDS USING FOKL-ASSISTED DIGESTION**

(57)  The present invention is in the field of biotechnology, in particular in the field of detection of pathogenic viruses, more particularly in the detection of single-stranded RNA (ssRNA) viruses. The present invention relates to an *in vitro* method for the detection of single-stranded nucleic acids, in particular ssRNA viruses, using the restriction enzyme Fokl. The invention also relates to a kit comprising the means necessary for the detection of these nucleic acids and its use for this purpose.

EP 4 253 560 A1

(52) Cooperative Patent Classification (CPC): (Cont.)

C-Sets
**C12Q 1/6818, C12Q 2521/301, C12Q 2525/301,
C12Q 2531/119;
C12Q 1/6844, C12Q 2521/301, C12Q 2525/301,
C12Q 2531/119**

**Description**

The present invention is in the field of biotechnology, in particular in the field of detection of pathogenic viruses, more particularly in the detection of single-stranded RNA (ssRNA) viruses. The present invention relates to an *in vitro* method for the detection of single-stranded nucleic acids, in particular ssRNA viruses, using the restriction enzyme FokI. The invention also relates to a kit comprising the means necessary for the detection of these nucleic acids and its use for this purpose.

**BACKGROUND ART**

The accurate detection of nucleic acids from certain biological pathogens is critical for the diagnosis of human diseases. Molecular approaches are powerful tools in pathogen discovery and aid in the diagnosis of human diseases. The early and accurate detection of detrimental pathogens has a dramatic effect on human wellbeing but is also associated with notable economic impacts worldwide.

Adequate signal quantification is a critical parameter for accurate pathogen diagnosis. As such, most classical approaches have relied on the use of the versatile molecular beacon technology. In order to amplify the detection signal of nucleic acid targets, several techniques have combined the use of different enzymatic activities with the design of specific molecular beacons, resulting in different isothermal amplification approaches. Some successful examples are nucleic acid sequence based amplification (NASBA, 3SR), loop mediated isothermal amplification (LAMP), rolling circle amplification (RCA) and nicking endonuclease signal amplification (NESA) methods, among others. While most of these approaches have proven to be useful in detecting target molecules, they all depend on the amplification of the nucleic acid content rather than on the amplification of the detection signal itself, and the enhanced detection is often hindered by the presence of high background levels caused by unwanted sources of amplification. On the other hand, signal amplification mediated by NESA does allow for the appropriate discrimination of DNA molecules in complex samples, and dramatically reduces the limit of detection as compared to other conventional approaches. However, direct detection of RNA molecules, by means of enhanced detection of DNA: RNA heteroduplexes, is still challenging due to the lack of knowledge about enzymatic activities that can induce symmetric cleavage of DNA: RNA hybrid strands.

The discovery of a set of restriction endonucleases (REs) able to hydrolyse DNA: RNA heteroduplexes paved the way for the development of novel approaches focused on the study of DNA: RNA hybrids. The robust and specific cleavage of both RNA and DNA strands has been achieved with at least 6 type IIP REs (AvaII, AvrII, BanI, HaeIII, HinfI and TaqI), despite the significant differences in the topology and sequence asymmetry caused by the DNA: RNA hybrid structure. FokI, a type of IIS RE can perform asymmetric digestion of DNA: RNA hybrids when the catalytic domain of the endonuclease is fused to specific Zinc Finger DNA-binding domains. FokI is an unusual restriction enzyme that recognizes a specific DNA sequence (GGATC) and cleaves non-specifically 9-13 bp away from the recognition site. In order to achieve the correct cleavage of both DNA strands, the dimerization of this endonuclease at the interface of the FokI cleavage domain is required. Second and third generation editing technologies, created from the combination of FokI cleavage domain with either Zinc Finger domains (Kim,Y.G., Cha,J. and Chandrasegaran,S. (1996) Hybrid restriction enzymes: zinc finger fusions to Fok I cleavage domain. Proc. Natl. Acad. Sci. U.S.A., 93, 1156-1160) or the recent CRISPR/Cas9 technology (Tsai,S.Q., et al. (2014) Dimeric CRISPR RNA-guided FokI nucleases for highly specific genome editing. Nat. Biotechnol., 32, 569-576) have also highlighted the potential of FokI as a powerful tool for genetic manipulation. In addition, a DNA/FokI-based replicating cutting machine was successfully used for the amplified signal detection of DNA analytes (Weizmann,Y., Cheglakov,Z., Pavlov,V. and Willner,I. (2006) An autonomous fueled machine that replicates catalytic nucleic acid templates for the amplified optical analysis of DNA. Nat Protoc, 1, 554-558), thus demonstrating the high versatility of this RE for the detection of particular DNA targets.

The recent pandemic of COVID19, caused by the novel human Betacoronavirus SARS-CoV-2, has highlighted the need for alternative scalable approaches or point-of-care systems to facilitate real-time disease surveillance.

The patent document US10815539 describes methods for detecting the presence of SARS-CoV-2 in a sample, including a clinical sample, and oligonucleotides, reagents and kits useful in such assays. In particular, the method comprises incubating the sample in the presence of (i) a DNA retrotranscriptase and a DNA polymerase with 5' to 3' exonuclease activity; (ii) an ORF1ab forward primer; (iii) an ORF1ab reverse primer; and a molecular probe (probe) of complementary sequence to ORF1ab (claim 8) or the S. The probe for the method can be a molecular beacon, although the authors express a preference for TaqMan probes. In addition, the method can be carried out using multiple primer pairs and multiple probes, designed for the detection of different regions of the viral genome.

The work of Ding X., etal. 2020. Nat Commun. describes an All-In-One Dual CRISPR-Cas12a (AIOD-CRISPR) assay for one-pot, ultrasensitive, and visual SARS-CoV-2 detection. By targeting SARS-CoV-2's nucleoprotein gene, two CRISPR RNAs without protospacer adjacent motif (PAM) site limitation are introduced to develop the AIOD-CRISPR assay and detect the nucleic acids with a sensitivity of few copies. The assay was validated by using COVID-19 clinical swab samples and obtain consistent results with RT-PCR assay. In the assay, simultaneous reverse transcription and isothermal amplification (RT-LAMP) reactions are carried out, followed by the detection and nicking of the virus target sequences by Cas12, with subsequent release of a fluorescent probe that binds specifically to single-stranded viral DNA.

The work of Kim JH. Et al., 2019. Biochip J. describes a simple colorimetric and multiplex loop-mediated isothermal amplification (LAMP) assay to rapid screening of severe acute respiratory syndrome-associated coronavirus. It can be visually detected based on colour change and monitored in real-time with fluorescent signals. The performance of this assay, based on six primers targeting open reading frame (ORF1b) and nucleocapsid (N) genes located in different regions of the SARS-CoV, was compared with real-time RT-PCR assay using various concentrations of target genes. Also, it describes a multiplex real-time LAMP assay to simultaneously detect two target regions within the SARS-CoV-2 genome.

The gold standard method for the clinical diagnosis of most pathogens is based on the use of the PCR technique. However, to avoid the collapse of the diagnostic capacity of the healthcare system under certain situations, such as in the COVID19 pandemic, the development of additional, rapid and inexpensive technologies for the detection of molecules with pathogenic potential for humans is essential.

Even though there are a plurality of documents describing different ways of detecting ssRNA viruses, in recent years mainly related to SARS-CoV-2, the amplified detection of RNA molecules from a complex sample by direct detection of RNA: DNA hybrids remains a challenge.

## DESCRIPTION OF THE INVENTION

The present invention is aimed to provide an alternative method for the detection of RNA and/or DNA molecules with pathogenic potential for humans and a valuable signal amplification technology that enables the detection of these molecules.

The authors of the present invention have developed a novel alternative molecular nucleic acid detection method based on the reaction driven by FokI, a classical restriction enzyme used in certain primigenial genome editing approaches.

The method described in the present invention reveals the unanticipated activity exerted by the IIS-type FokI endonuclease in the context of DNA duplexes and DNA:RNA hybrids and highlights the possibility of considering this reaction as a potential diagnostic method for the identification of viral pathogens of particular relevance for human health.

Thus, in a first aspect, the invention relates to an *in vitro* method for detecting the presence of at least one single-stranded nucleic acid, (hereinafter the "method of the invention") in an isolated sample from a subject, wherein said method comprises:

(I) incubating the sample in the presence of a FokI endonuclease and at least one molecular beacon, wherein said beacon comprises:

a) a double-stranded DNA fragment comprising a FokI recognition site;
b) an oligonucleotide complementary to a nucleic acid region of the single-stranded nucleic acid;
c) a fluorophore at one of the ends, preferably at the 5' end; and
d) a fluorescence quencher molecule at the end opposite to the end of (c), preferably at the 3' end;

(II) determine whether said single-stranded nucleic acid is present in the sample by determining whether a fluorescent signal of said fluorophore has become detectable.

The incubation of the sample in the presence of a FokI endonuclease and at least one molecular beacon is carried out under conditions that allow:

(i) specific hybridization of the beacon to the nucleic acid region of the single-stranded nucleic acid of (b); and
(ii) the unique cleavage of the beacon by the FokI endonuclease and the consequent release of the original nucleic acid region of (b) and the fluorophore of (c), allowing the fluorescent signal generated to be detectable.

In a preferred embodiment of the method of the invention, the single-stranded nucleic acid is a single-stranded virus, a single-stranded DNA (ssDNA) virus or a single-stranded RNA (ssRNA) virus, more preferably a ssRNA virus.

As used herein, the term "detect" or "detecting" refers to report or identify the presence of at least one single-stranded nucleic acid, preferably a ssDNA virus or a ssRNA virus, more preferably a ssRNA virus, that are present in the sample by generating a fluorescent signal.

The term "*in vitro*" means that the detection of the single-stranded nucleic acid is performed outside subject's body.

Molecular beacons are hairpin-shaped oligonucleotide probes that fluoresce in the presence of a target RNA or DNA sequence. The structure of these molecular beacons is determined by the presence of a fluorophore at one end and a quencher at the other end of the probe. The term "quencher" refers to a fluorescence inhibitor molecule.

By using restriction nicking enzymes or nickases, it is possible to digest the molecular beacons themselves bound to their recognition sequence in the presence of DNA duplexes and RNA: DNA hybrids. This phenomenon allows the release of the inhibited fluorophore and, at the same time, an exponential amplification of the signal, considerably reducing the limits of detection of exotic molecules in the sample, such as viral RNA in samples from infected patients.

FokI is an IIS-type restriction endonuclease (RE). FokI is an unusual restriction enzyme that recognizes a specific DNA sequence (GGATC) and cleaves non-specifically 9-13 bp away from the recognition site.

FokI can be used to carry out a "Basic FokI-assisted signal amplification assay", which is performed like conventional hybridization but including the addition of FokI and/or for an "Enhanced FokI signal amplification assay", which is performed like basic FokI-assisted signal amplification but is performed in the presence of universal hairpin oligonucleotide. This universal hairpin is labelled with a fluorophore and a quencher at its 5' and 3' ends, respectively. The term "hybridization" refers to the process of combining two complementary single-stranded DNA or RNA molecules and allowing them to form a single double-stranded molecule through base pairing.

In a conventional hybridization assay, the absence of the complementary target molecule causes a Förster resonance energy transfer (FRET), which inhibits the fluorescence of the molecular beacon due to the proximity of the fluorescent dye-quencher pair. However, in the presence of the target sequence, one molecule of nucleic acid substrate can hybridize to a molecule of the molecular beacons generating a duplex/heteroduplex substrate and releasing the fluorescent signal of the reporter molecule. The implementation of the FokI-assisted digestion system, using the combination of molecular beacons and native FokI, improves the detection of nucleic acids, as the cleavage reaction and the subsequent release of the fluorescent signal depend on the hybridization between these oligonucleotides and the target sequence. The asymmetric cleavage performed by FokI in this context could allow for the rapid release of the fluorescent portion of the probe and the potential recirculation of the target sequence for further rounds of digestion.

In Enhanced FokI signal amplification the by-products from the FokI-assisted reaction can still be used for further rounds of digestion in the presence of universal hairpin oligonucleotides. These universal hairpin oligonucleotides are labelled with fluorescent dyes and quencher molecules at their 5' and 3' ends, respectively, and contain an internal FokI recognition site and a 5' end overhang that can hybridize with the resulting product of the FokI-assisted reaction. The resulting substrate can be further digested by circulating FokI units, releasing the fluorescent part of the universal hairpin oligonucleotide and creating two different subproducts. As one of the subproducts still retains a FokI recognition site and a 5' overhang which can hybridize with the remaining universal hairpin oligonucleotides, the new set of actionable substrates can be further digested by FokI, generating the aforementioned subproducts and creating a self-sustained loop for signal amplification purposes.

In a particular embodiment, the FokI-assisted signal amplification is carried out for 25-90 minutes (both ends included), preferably for 45 minutes, and at a temperature between 30-40ºC (both ends included), preferably at 37ºC.

In another particular embodiment, the fluorophore of the molecular beacon, which is preferably at the 5' end of the hairpin, is FAM, HEX, TET, JOE, VIC, Cy3, NED, TAMRA, Cy3.5, ROC, Texas Red or Cy5, preferably FAM or HEX. FAM has an absorption maximum of 488 nm and an emission maximum of 520 nm; HEX has an absorption maximum of 535 nm and an emission maximum of 556 nm; TET has an absorption maximum of 521 m and an emission maximum of 536 nm, JOE has an absorption maximum of 529 nm and an emission maximum of 555 nm; VIC has an absorption maximum of 538 nm and an emission maximum of 554 nm; Cy3 has an absorption maximum of 549 nm and an emission maximum of 566 nm and NED has an absorption maximum of 546 nm and an emission maximum of 575 nm; TAMRA has an absorption maximum of 557 nm and an emission maximum of 583 nm; Cy3.5 has an absorption maximum of 581 nm and an emission maximum of 596 nm; ROX has an absorption maximum of 586 nm and an emission maximum of 610 nm; Texas Red has an absorption maximum of 597 nm and an emission maximum of 616 nm and Cy5 has an absorption maximum of 646 nm and an emission maximum of 669 nm.

In another particular embodiment, the quencher of the molecular beacon, which is preferably at the 3' end of the hairpin, is BHQ1 or BHQ2, preferably BHQ1. BHQ quenchers have broad absorption spectra and can be paired with reporter dyes that emit in different ranges. BHQ1 is typically used to quench in the range 480 - 580 nm and BHQ2 is used to quench in the range 559-670 nm.

In a particular embodiment, the fluorophore-quencher pair of the molecular beacon is selected from the list: FAM-BHQ1, HEX-BHQ1, TET-BHQ1, JOE-BHQ1, VIC-BHQ1, Cy3-BHQ1, Cy3-BHQ2, NED-BHQ1, NED-BHQ2, TAMRA-BHQ2, Cy3.5-BHQ2, ROX-BHQ2, Texas Red-BHQ2 or Cy5-BHQ2, preferably FAM-BHQ1 or HEX-BHQ1.

As mentioned at the beginning of this description, the single-stranded nucleic acid is, preferably, a single-stranded DNA (ssDNA) virus or a single-stranded RNA (ssRNA) virus, more preferably a ssRNA virus.

Thus, in a particular embodiment, the ssRNA virus is one that causes nasal and/or oral infections or has an oral and/or nasal presence in the subject.

In another particular embodiment, the ssRNA virus is selected from the virus families: Orthomyxoviridae, Coronaviridae, Picornaviridae, Paramyxoviridae and Pneumoviridae. Preferably, the ssRNA virus is selected from the list consisting of: *Influenza A, Influenza B* and *Influenza* C (family Orthomyxoviridae), *Alphacoronavirus* and *Betacoronavirus* (family Coronaviridae), *Rhinovirus* and *Parechovirus* (family Picornaviridae), *Respirovirus* and *Rubulavirus* (family Paramyxoviridae), *Metapneumovirus* and *Orthopneumovirus* (family Pneumoviridae).

Throughout this document the term Parainfluenza virus will be used to refer to the *Respirovirus* and *Rubulavirus* genera; and *Pneumovirus* will be used to refer to the *Metapneumovirus* and *Orthopneumovirus* genera.

In a more particular embodiment, the ssRNA virus is selected from: *Influenza A* virus subtypes H1N1, H2N2, H3N2 and H7N9; *Alphacoronavirus* species 229E and NL63; *Betacoronavirus* specie HKU1, strain OC43 and SARS-CoV-2; the human respiroviruses type 1 (HPIV-1) and type 3 (HPIV-3); the human rubulavirus type 2 (HPIV-2) and type 4 (HPIV-4); the Human Metapneumovirus (HMPV) or the human respiratory syncytial virus (hRSV).

The molecular beacons that are designed to detect the presence of at least one ssRNA virus comprise an oligonucleotide complementary to an RNA region of the ssRNA virus, which will allow specific hybridization of the molecular beacon to the RNA region of the ssRNA virus. In a preferred embodiment, the RNA region selected of *Influenza A* virus (H1N1, H2N2, H3N2 or H7N9), *Influenza B* virus and/or *Influenza C* virus, is the nucleoprotein. In the case of *Alphacoronavirus* (229E or NL63), the RNA region is Nsp13. The RNA region of *Betacoronavirus* (OC43, HKU1) is the nucleocapsid and, in the case of SARS-CoV-2, the RNA region can be the Spike protein or the Orf8 protein. For *Rhinovirus* and *Parechovirus* the RNA region is the 5'UTR region; and, in the case of *Respirovirus* (HPIV1 or HPIV3), *Rubulavirus* (HPIV2 or HPIV4), *Metapneumovirus* (HMPV) and/or *Orthopneumovirus* (hRSV) the RNA region is RdRP-L (Table 1).

Therefore, the RNA region of the ssRNA virus is selected from those coding for: the *Influenza* virus nucleoprotein, the Nsp13 protein of *Alphacoronavirus,* the nucleocapsid of *Betacoronavirus,* the Spike protein or the Orf8 protein of SARS-CoV-2, the 5'UTR region of *Rhinovirus* and *Parechovirus,* the RdRP-L of *Respirovirus, Rubulavirus, Metapneumovirus, Orthopneumovirus* and/or any combination thereof.

The sequences of the molecular beacons that can be used in the method of the invention for detecting the presence of at least one ssRNA virus comprise, preferably consist of, SEQ ID NO:1-11 (Table 1). The sequences of the molecular beacons (MB) that hybridize to the nucleoprotein of *Influenza* virus, comprise, preferably consist of, SEQ ID NO: 1-3 (SEQ ID NO: 1 for Influenza A, SEQ ID NO: 2 for Influenza B and SEQ ID NO: 3 for Influenza C). The sequence of the molecular beacon that hybridizes to the Nsp13 protein of *Alphacoronavirus* comprises, preferably consists of, the SEQ ID NO: 4. The sequence of the molecular beacon that hybridizes to the nucleocapsid of *Betacoronavirus* comprises, preferably consists of, the SEQ ID NO: 5. The sequences of the molecular beacons that hybridize to SARS-CoV-2 comprise, preferably consist of, SEQ ID NO: 6-7 (SEQ ID NO: 6 for Spike protein and SEQ ID NO: 7 for Orf8 protein). The sequences of the molecular beacons that hybridize to the 5'UTR region of *Rhinovirus* and *Parechovirus* comprise, preferably consist of, SEQ ID NO: 8-9, respectively. The sequences of the molecular beacons that hybridize to the RdRP-L of the Family Paramyxoviridae and Pneumoviridae comprise, preferably consist of, SEQ ID NO: 10-11, respectively.

*Influenza A virus,* Class V (ss -) in Baltimore classification, causes influenza in birds and some mammals and is the only species of the genus *Alphainfluenzavirus* of the virus family Orthomyxoviridae. Influenza A viruses are negative-sense, single-stranded, segmented RNA viruses. "Human influenza virus" usually refers to those subtypes that spread widely among humans. H1N1, H1N2, and H3N2 are the only known influenza A virus subtypes currently circulating among humans. The subtype H7N9 is a bird flu strain.

*Influenza B virus,* Class V (ss -) in Baltimore classification, is the only species in the genus *Betainfluenzavirus* in the virus family Orthomyxoviridae. Influenza B virus is known only to infect humans and seals. There are two known circulating lineages of Influenza B virus based on the antigenic properties of the surface glycoprotein hemagglutinin. *Influenza B virus* mutates at a rate 2 to 3 times slower than type A. Influenza B viruses are negative-sense, single-stranded RNA viruses.

*Influenza C virus,* Class V (ss-) in Baltimore classification, is the specie in the genus *Gammainfluenzavirus,* in the virus family Orthomyxoviridae, which like other influenza viruses, causes influenza. Influenza C viruses are known to infect humans and pigs. Flu due to the Type C species is rare compared to Types B or A but can be severe and can cause local epidemics. Type C has 7 RNA segments and encodes 9 proteins, while Types A and B have 8 RNA segments and encode at least 10 proteins. *Influenza A virus* is the most severe, *Influenza B virus* is less severe but can still cause outbreaks, and *Influenza C virus* is usually only associated with minor symptoms. Influenza C viruses are negative-sense, single-stranded RNA viruses.

The nucleoprotein (NP) of Influenza viruses covers the viral RNA entirely and it is this NP-RNA complex that is the template for transcription and replication by viral polymerase.

Alphacoronaviruses, Class IV (ss +) in Baltimore classification, are positive-sense, single-stranded RNA viruses that infect mammals, including humans. They have spherical virions with club-shaped surface projections formed by trimers of the spike protein, and a viral envelope. Alphacoronaviruses are in the subfamily Orthocoronavirinae of the family Coronaviridae. 229E and NL63 are two of the seven coronaviruses known to infect humans. Nonstructural protein 13 (Nsp13), the RNA region selected for *Alphacoronavirus,* separates the double-stranded replicative intermediates to provide single-stranded templates for RNA synthesis.

Betacoronaviruses, Class IV (ss +) in Baltimore classification, are enveloped, positive-sense, single-stranded RNA viruses that infect mammals (of which humans are part). The genus is in the subfamily Orthocoronavirinae in the family Coronaviridae, of the order Nidovirales. The betacoronaviruses of the greatest clinical importance concerning humans are OC43 and HKU1 (which can cause the common cold) of lineage A, SARS-CoV and SARS-CoV-2 (which causes the disease COVID-19) of lineage B, and MERS-CoV of lineage C. The nucleocapsid (N) protein, the RNA region selected for OC43 and HKU1, is a protein that packages the positive-sense RNA genome of coronaviruses to form ribonucleoprotein structures enclosed within the viral capsid.

Severe acute respiratory syndrome coronavirus type 2, abbreviated SARS-CoV-2 (severe acute respiratory syndrome coronavirus 2) or SARS-CoV-2 is a type of coronavirus that causes the 2019 coronavirus disease. Is in the Coronaviridae family, genus *Betacoronavirus,* subgenus *Sarbecovirus,* species *SARS virus.* The virus genome consists of a single strand of RNA and is classified as a positive single-stranded RNA virus. Spike (S) glycoprotein is the largest of the four major structural proteins found in coronaviruses. The function of the spike glycoprotein is to mediate viral entry into the host cell by first interacting with molecules on the exterior cell surface and then fusing the viral and cellular membranes. Orf8 is a rapidly evolving accessory protein that has been proposed to interfere with immune response. ORF8 is a 121-amino acid (aa) protein consisting of an N-terminal signal sequence followed by a predicted Ig-like fold.

*Rhinovirus,* Class IV (ss +) in Baltimore classification, is the most common viral infectious agent in humans and is the predominant cause of the common cold. Rhinoviruses belong to the genus *Enterovirus* in the family Picornaviridae. Rhinoviruses have single-stranded positive sense RNA genomes of between 7200 and 8500 nt in length. The three species of rhinovirus (A, B, and C) include around 160 recognized types of human rhinovirus that differ according to their surface proteins (serotypes). The primary route of entry for human rhinoviruses is the upper respiratory tract (mouth and nose).

*Parechovirus,* Class IV (ss +) in Baltimore classification, is a genus of viruses in the family Picornaviridae. Humans, ferrets, and various rodents serve as natural hosts. Parechoviruses have single-stranded positive sense RNA genomes.

The region 5'-UTR of the virus of the Family Picornaviridae (*Rhinovirus* and *Parechovirus*) can form complex structures, such as stem-loop, clover and pseudoknot structure. These structures play an important role in the regulation of the replication and translation of the viruses.

Parainfluenza viruses, Class V (ss -) in Baltimore classification, are a set of paramyxoviruses about 100-200 nanometres in size, with a nucleocapsid diameter of 18 nm, enveloped by two glycoproteins. Human parainfluenza viruses (HPIVs) are the viruses that cause human parainfluenza. Parainfluenza viruses comprises the *Respirovirus* and *Rubulavirus* genera. *Respirovirus* is a genus of viruses in the order Mononegavirales, in the family Paramyxoviridae. Rodents and human serve as natural hosts. There are seven species in this genus. The human respiroviruses HPIV-1 and HPIV-3 are distributed worldwide and are an important cause of respiratory tract diseases in children. *Rubulavirus* is a genus of viruses in the order Mononegavirales, in the family Paramyxoviridae. HPIV2 and HPIV4 cause acute laryngotracheitis in children and respiratory disease in humans, respectively.

Pneumoviruses, Class V (ss -) in Baltimore classification, are any virus of a family (Pneumoviridae) of single-stranded RNA viruses that have a filamentous or spherical virion with a lipid-containing envelope, typically cause respiratory infections in mammals and birds, include the respiratory syncytial virus and metapneumovirus. The genus *Orthopneumovirus* consists of pathogens that target the upper respiratory tract within their specific hosts. Orthopneumoviruses are found among sheep, cows, and most importantly humans. In humans, the orthopneumovirus that specifically impacts infants and small children is known as human respiratory syncytial virus. *Metapneumovirus* may cause respiratory disease of varying severity, especially in children.

The viral RNA-dependent RNA polymerase (RdRP), the RNA region selected for Pneumoviruses, is composed of the L and P proteins. Catalytic activities for RNA polymerization, capping, and polyadenylation reside within the L protein.

Table 1 lists the single-stranded (ssRNA) viruses, that can be detected by the method of the invention, as well as the sequences of the molecular beacons (SEQ ID NO: 1-11) that can be used in the method of the invention.

**Table 1.** Table of ssRNA viruses that can be detected by the method of the invention.

| Family | Target Organism | | Target Region | Oligo Sequence (MB) |
|---|---|---|---|---|
| Orthomyxoviridae | Influenza A | H1N1, H2N2, H3N2, H7N9 | NP | SEQ ID NO: TCTTCGGAGACAAT GCAGACGAAGAGTC ATCCGACGTCGGAT GAC 1 |
| | Influenza B | - | NP | SEQ ID NO: TGTGACTGTTTCAG TACGTGTCACAGTC ATCCGACGTCGGAT GAC 2 |
| | Influenza C | - | NP | SEQ ID NO: AGTGCTTTCTCCCTT ATTGAGCACTGTCA TCCGACGTCGGATG AC 3 |
| Coronaviridae | Alphacoronavirus | 229E, NL63 | Nsp13 | SEQ ID NO: GAGACTAAATGATT ACTAAAAGTCTCGT CATCCGACGTCGGA TGAC 4 |
| | Betacoronavirus | OC43, HKU1 | Nucleocapsid | SEQ ID NO: AGCAGACCTTCCTG AGCCTTCTGCTGTC ATCCGACGTCGGAT GAC 5 |
| | | SARS-CoV-2 | Spike | SEQ ID NO: TGCTGATTCTCTTCC TGTTTCAGCAGTCA TCCGACGTCGGATG AC 6 |
| | | | Orf8 | SEQ ID NO: TGCTGATTTTCTAGC TCCTTCAGCAGTCA TCCGACGTCGGATG AC 7 |

(continued)

| Family | Target Organism | Target Region | Oligo Sequence (MB) |
|---|---|---|---|
| Picornaviridae | *Rhinovirus* | Multiple (>110) | 5'UTR | SEQ ID NO: ACCCAAAGTAGTTG GTCCCTTGGGTGTC ATCCGACGTCGGAT 8GAC |
| | *Parechovirus* | 1-8 | 5'UTR | SEQ ID NO: GTTACCTACGGGTA CCTTCGGTAACGTC ATCCGACGTCGGAT 9 GAC |
| Paramyxoviridae | *Respirovirus* | HPIV1 and HPIV3 | RdRP-L | SEQ ID NO: TGGTTCAAGGAGAC AATCAGAACCAGTC ATCCGACGTCGGAT 10 GAC |
| | *Rubulavirus* | HPIV2 and HPIV4 | | |
| Pneumoviridae | *Metapneumovirus* | HMPV | | SEQ ID NO: GGTAGAATGTTTGC 11 TATGCTCTACCGTC |
| | *Orthopneumovirus* | hRSV | RdRP-L | |
| | | | | ATCCGACGTCGGAT GAC |

The method of the invention can be performed on any sample that is susceptible to contamination by a single-stranded nucleic acid, preferably ssRNA or ssDNA viruses, more preferably ssRNA viruses. The term "sample" refers to a small part or quantity of a thing which is considered representative of the whole and which is taken or separated from it for the purpose of study, analysis or experimentation. In the present invention, said study, analysis or experimentation refers to the detection of the presence of at least one single-stranded nucleic acid, preferably a ssDNA virus or a ssRNA virus, more preferably a ssRNA virus. The term "sample" also includes samples that have been manipulated in some way after their collection e.g., by treatment with reagents, solubilisation or enrichment of certain components. In a preferred embodiment, the sample is a biological sample.

The term "biological sample" includes, but is not limited to, biological tissues and/or fluids from an individual, obtained by any method known to a person skilled in the art for that purpose. In a more preferred embodiment, the biological sample is isolated from the oral or respiratory tract, most preferably, by means of a nasopharyngeal swab.

The sample is isolated from a subject. The term "subject" as used in the present invention refers to any animal, preferably a mammal. In a more preferred embodiment, the subject is a human being of any sex, age or race.

In particular embodiment, the method of the invention also includes a previous amplification step, preferably an isothermal amplification, more preferably a rolling circle amplification (RCA). The RCA technique relies on the isothermal amplification of circular DNA targets mediated by the high processivity and strand displacement activity of the Phi29 DNA polymerase. The RCA reaction involves the use of padlock probes. The term "probe" means a DNA fragment used to detect the presence of a specific nucleic acid within a sample through hybridization. The probes can be produced by the methods known to a person skilled in the art. For example, they can be produced by chemical synthesis.

Padlock probes are probes which contain a phosphate modification at their 5' end. The hybridization of a target molecule with a padlock probe reorganizes the configuration of the padlock probe, which adopts a circular structure. The catalytic activity of a DNA ligase enzyme facilitates the full circularization of the padlock probe by ligating its splinted 5' and 3' ends. The resulting single stranded DNA template can be used for indefinite rounds of amplification in the presence of a complementary RCA primer, dNTPs and the Phi29 DNA polymerase, thus increasing the number of target sequences in the reaction mixture. Padlock probes that can be used in the amplification step of the method of the invention comprise, preferably consist of, SEQ ID NO: 34-44 (Table 2). Padlock probes for the amplification of *Influenza A, Influenza B* or *Influenza* C virus comprise, preferably consist of, SEQ ID NO: 34-36 (SEQ ID NO: 34 for Influenza A, SEQ ID NO: 35 for Influenza B and SEQ ID NO: 36 for Influenza C). The padlock probe for the amplification of *Alphacoronavirus* comprises, preferably consists of, the SEQ ID NO: 37. The padlock probe for the amplification of *Betacoronavirus* comprises, preferably consists of, the SEQ ID NO: 38. Padlock probes for the amplification of SARS-CoV-2 comprise, preferably consist of, SEQ ID NO: 39-40 (SEQ ID NO: 39 for the Spike protein and SEQ ID NO: 40 for Orf8 protein). The padlock probe for the amplification of *Rhinovirus* comprises, preferably consists of, the SEQ ID NO: 41. The padlock probe for the amplification of *Parechovirus* comprises, preferably consists of, the SEQ ID NO: 42. The padlock probe for the amplification of Parainfluenza virus comprises, preferably consists of, the SEQ ID NO: 43. The padlock probe for the amplification of *Pneumovirus* comprises, preferably consists of, the SEQ ID NO: 44.

The molecular coupling of the FokI-assisted signal amplification assay improves on the detection results obtained in classical RCA approaches. Target molecules can hybridize with the molecular beacons and generate a duplex/heteroduplex substrate that can release the fluorescent signal of the reporter molecule. The asymmetric cleavage reaction mediated by FokI can enhance the excision of the fluorescent portion of the probe and the potential reuse of the target sequence or further rounds of digestion, thus improving the detection signal of reaction. The presence of the FokI endonuclease in the reaction mixture greatly outperform the response of the fluorescence signal alone and reduced the detection time as compared with classical RCA approach.

The RCA - FokI-assisted signal amplification reaction is performed in two subsequent steps. First, padlock probes containing a phosphate modification at their 5' end are hybridized with their corresponding RNA molecules and the padlock probe circularization step is performed in the presence of a DNA ligase, preferably the SplintR ligase. Incubation of these padlock probes with their RNA targets and the SplintR DNA ligase led to successful circularization in the presence of their corresponding target molecules, but not with unrelated RNA sequences. Then, an RCA premix was added to the resulting reaction. The RCA premix comprises: the universal rolling circle primer (SEQ ID NO: 45, Table 2), dNTPs, Phi29 DNA polymerase or a form of the Phi29 DNA polymerase (e.g., QualiPhi DNA polymerase), Pyrophosphatase Inorganic from *E. coli,* primers, at least one molecular beacon and a FokI RE.

Phi29 DNA polymerase is the replicative polymerase from the *Bacillus subtilis* phage phi29 (Φ29). This polymerase has exceptional strand displacement and processive synthesis properties. The polymerase has an inherent 3'→5' proofreading exonuclease activity. The expression "a form of the Phi29 DNA polymerase" refers to a Phi29 DNA polymerase derived from the Phi29 DNA polymerase or to a version of the Phi29 DNA polymerase (e.g., the QualiPhi DNA polymerase). QualiPhi DNA polymerase is a novel, highly processive chimeric form of Phi29 DNA polymerase engineered for improved sensitivity and efficiency by fusing DNA binding domains to the C terminus of the polymerase.

The term "primer" refers to an oligonucleotide capable of acting as a starting point for DNA synthesis when hybridized to the template nucleic acid. Primers can be prepared by any suitable method, including, for example, but not limited to, cloning and restriction of appropriate sequences and direct chemical synthesis. Primers can be designed to hybridize to specific nucleotide sequences in the template nucleic acid (specific primers), as in the present invention, or can be synthesised at random (arbitrary primers).

The DNA ligase is a specific type of enzyme, a ligase, that facilitates the joining of DNA strands together by catalyzing the formation of a phosphodiester bond. It plays a role in repairing single-strand breaks in duplex DNA in living organisms, but some forms (such as DNA ligase IV) may specifically repair double-strand breaks (i.e., a break in both complementary strands of DNA). In the present invention, the catalytic activity of a DNA ligase enzyme facilitates the full circularization of the padlock probe by ligating its splinted 5' and 3' ends. In a preferred embodiment, the DNA ligase used in the method of the invention is SplintR DNA ligase. SplintR ligase, also known as PBCV-1 DNA ligase or *Chlorella* virus DNA ligase, efficiently catalyzes the ligation of adjacent, single-stranded DNA splinted by a complementary RNA strand.

The term dNTP refers to deoxyribose nucleotides (dATP, dGTP, dCTP and dTTP). They are the building blocks of DNA. dNTPs are used to synthesize DNA.

In a particular embodiment, the RCA reaction is carried out during less than 70 minutes and at a temperature between 30-40ºC, preferably for 60 minutes at 37ºC.

Table 2 lists of primers, molecular beacons (SEQ ID NO: 1-11) and padlock probes (SEQ ID NO: 34-44) that can be used in the method of the invention for the detection of ssRNA viruses in an isolated sample.

**Table 2.** List of primers, molecular beacons and padlock probes that can be used in the method of the invention.

| Name | Target region | | Sequence |
|---|---|---|---|
| *Influenza A* | NP | MB | TCTTCGGAGACAATGCAGACGAAGA<br>SEQ ID NO:1 GTCATCCGACGTCGGATGAC |
| | | DNA | TCGTACTCCTCTGCATTGTCTCCGAA<br>SEQ ID NO: 12 GAAA |
| | | RNA | SEQ ID NO:<br>UCGUACUCCUCUGCAUUGUCUCCGA<br>23 AGAAA |
| | | Padlock probe | SEQ ID NO: 34<br><br>5P-<br>GCAGAGGAGTACGACATTATCCCTAT<br>AGTGAGTCGTATTAGAATTTTCCCAG<br>TCGTACGCAATTACTTCGGAGACAAT |
| *Influenza B* | NP | MB | SEQ ID NO:<br>TGTGACTGTTTCAGTACGTGTCACAG<br>2 TCATCCGACGTCGGATGAC |
| | | DNA | ATCCCAAACGTACTGAAACAGTCACA<br>SEQ ID NO: 13 GCCA |
| | | RNA | AUCCCAAACGUACUGAAACAGUCAC<br>SEQ ID NO: 24 AGCCA |
| | | Padlock probe | SEQ ID NO:<br>5P-<br>TCAGTACGTTTGGGATATTATCCCTA<br>TAGTGAGTCGTATTAGAATTTTCCCT<br>35 GGCATGAAGAATTGGCTGTGACTGTT |
| *Influenza C* | NP | MB | AGTGCTTTCTCCCTTATTGAGCACTG<br>SEQ ID NO: 3 TCATCCGACGTCGGATGAC |
| | | DNA | SEQ ID NO:<br>GAATCTCAATAAGGGAGAAAGCACTG<br>14 TTGA |
| | | RNA | GAAUCUCAAUAAGGGAGAAAGCACU<br>SEQ ID NO: 25 GUUGA |
| | | Padlock probe | 5P-<br>CTTATTGAGATTCACAATTATCCCTAT<br>AGTGAGTCGTATTAGAATTTTCCCAG<br>SEQ ID NO: 36 TCGTTCAAACAACAGTGCTTTCTCC |

(continued)

| Name | Target region | | Sequence |
|---|---|---|---|
| *Alphacorona virus* | Nsp13 | MB | GAGACTAAATGATTACTAAAAGTCTC SEQ ID NO: 4 GTCATCCGACGTCGGATGAC |
| | | DNA | SEQ ID NO: 15 TTGAGTTTTAGTAATCATTTAGTCTCA ACTAA |
| | | RNA | UUGAGUUUUAGUAAUCAUUUAGUCU SEQ ID NO: 26 CAACUAA |
| | | Padlock probe | SEQ ID NO: 5P- TACTAAAACTCAAACAATTATCCCTAT AGTGAGTCGTATTAGAATTTTCCCTTA 37 GGTACGCAGTTGAGACTAAATGAT |
| *Betacoronavirus* | Nucleocapsid | MB | AGCAGACCTTCCTGAGCCTTCTGCTG SEQ ID NO:5 TCATCCGACGTCGGATGAC |
| | | DNA | GTTGAAGGCTCAGGAAGGTCTGCTT SEQ ID NO: 16 CTAAT |
| | | RNA | GUUGAAGGCUCAGGAAGGUCUGCU SEQ ID NO: 27 UCUAAU |
| | | Padlock probe | SEQ ID NO: 5P- CCTGAGCCTTCAACATATTATCCCTA TAGTGAGTCGTATTAGAATTTTCCCA 38 GTCGTACGCAATTAGAAGCAGACCTT |
| SARS-CoV-2 | Spike | MB | TGCTGATTCTCTTCCTGTTTCAGCAG SEQ ID NO: 6 TCATCCGACGTCGGATGAC |
| | | DNA | CTTGGAACAGGAAGAGAATCAGCAA SEQ ID NO: 17 CTGTG |
| | | RNA | CUUGGAACAGGAAGAGAAUCAGCAA SEQ ID NO: 28 CUGUG |
| | | Padlock probe | 5P- GTTCCAAGCATAAACAATTATCCCTAT SEQ ID NO: 39 |

(continued)

| Name | Target region | | Sequence |
|---|---|---|---|
| | | | AGTGAGTCGTATTAGAATTTTCCCAG TCGTACGCATGCTGATTCTCTTCCT |
| | Orf8 | MB | TGCTGATTTTCTAGCTCCTTCAGCAG SEQ ID NO: 7 TCATCCGACGTCGGATGAC |
| | | DNA | AGAGTAGGAGCTAGAAAATCAGCAC SEQ ID NO: 18 CTTTA |
| | | RNA | AGAGUAGGAGCUAGAAAAUCAGCAC SEQ ID NO: 29 CUUUA |
| | | Padlock probe | 5P- CCTACTCTAATATACCATTATCCCTAT AGTGAGTCGTATTAGAATTTTCCCAG SEQ ID NO: 40 TCGTACGCATGCTGATTTTCTAGCT |
| Rhinovirus | 5'UTR | MB | ACCCAAAGTAGTTGGTCCCTTGGGTG SEQ ID NO: 8 TCATCCGACGTCGGATGAC |
| | | DNA | CGGGATGGGACCAACTACTTTGGGT SEQ ID NO: 19 GTCCG |
| | | RNA | CGGGAUGGGACCAACUACUUUGGG SEQ ID NO: 30 UGUCCG |
| | | Padlock probe | SEQ ID NO: 5P- GTCCCGTCCCATCCCGATTATCCCTA TAGTGAGTCGTATTAGAATTTTCCCA 41 GTCGTACGGAACACCCAAAGTAGTTG |
| Parechovirus | 5'UTR | MB | GTTACCTACGGGTACCTTCGGTAACG SEQ ID NO: 9 TCATCCGACGTCGGATGAC |
| | | DNA | TGCCCAGAAGGTACCCGTAGGTAAC SEQ ID NO: 20 AAGTG |
| | | RNA | SEQ ID NO: 31 UGCCCAGAAGGUACCCGUAGGUAAC AAGUG |
| | | Padlock probe | 5P- TACCTTCTGGGCATCCATTATCCCTA TAGTGAGTCGTATTAGAATTTAGCAT ATCCATACCAACTTGTTACCTACGGG SEQ ID NO: 42 |

(continued)

| Name | Target region | Sequence | | |
|---|---|---|---|---|
| Parainfluenza | RdRP-L | MB | TGGTTCAAGGAGACAATCAGAACCAG TCATCCGACGTCGGATGAC | SEQ ID NO: 10 |
| | | DNA | ATAGCTTGATTGTCTCCTTGAACCATT GC | SEQ ID NO: 21 |
| | | RNA | AUAGCUUGAUUGUCUCCUUGAACCA UUGC | SEQ ID NO: 32 |
| | | Padlock probe | 5P- GACAATCAAGCTATAGATTATCCCTA TAGTGAGTCGTATTAGAATTTTCCCA GTCGTACGGATGCAATGGTTCAAGG A | SEQ ID NO: 43 |
| Pneumovirus | RdRP-L | MB | GGTAGAATGTTTGCTATGCTCTACCG TCATCCGACGTCGGATGAC | SEQ ID NO: 11 |
| | | DNA | TTCCTGGTTGCATAGCAAACATTCTA CCTA | SEQ ID NO: 22 |
| | | RNA | UUCCUGGUUGCAUAGCAAACAUUCU ACCUA | SEQ ID NO: 33 |
| | | Padlock probe | 5P- GCTATGCAACCAGGAAATTATCCCTA TAGTGAGTCGTATTAGAATTATGCCA GTCGTACGGATGTAGGTAGAATGTTT | SEQ ID NO: 44 |
| Universal RCA primer | | SEQ ID NO: 45 AATTCTAATACGACTCACTATAGG^G^ | | |
| ^ = 3' - Phosphonothioate modification | | | | |

In another particular embodiment, the method of the invention can be used to detect, in the isolated sample, the presence of more than one single-stranded nucleic acid, preferably more than one ssDNA or ssRNA virus, or more than one region of the same single-stranded nucleic acid, wherein more than one molecular beacon is used, each comprising a different oligonucleotide complementary to a different region of the same single-stranded nucleic acid or a different oligonucleotide complementary to a different single-stranded nucleic acid.

In a preferred embodiment, the method of the invention can be used to detect, in the isolated sample, the presence of more than one ssRNA virus or more than one RNA region of the same ssRNA virus, wherein more than one molecular beacon is used, each comprising a different oligonucleotide complementary to a different RNA region of the same ssRNA virus or a different oligonucleotide complementary to a different ssRNA virus.

In a more preferred embodiment, the ssRNA virus is SARS-CoV-2, preferably wild type and/or any of its variants, more preferably the strains B.1.1.7, B.1.351, P.1 and/or B.1.617.2.

Strain B.1.1.7 is characterized by having the mutation N501Y, strain B.1.351 is characterized by having N501Y, E484K and K417N mutations, strain P.1 is characterized by having N501Y, E484K and K417T mutations and strain B.1.617.2 is characterized by having L452R, T478K and P681R mutations.

The "N501Y" mutation refers to a mutation in amino acid 501 of the S protein of the virus in which there is a change from asparagine (N) to tyrosine (Y). The "E484K" mutation refers to a mutation in amino acid 484 of the S protein of the virus in which there is a change from glutamic acid (E) to lysine (K). The "K417N" mutation refers to a mutation in amino acid 417 of the S protein of the virus in which there is a change from lysine (K) to asparagine (N). The " L452R" mutation refers to a mutation in amino acid 452 of the S protein of the virus in which there is a change from leucine (L) to arginine (R). The " T478K" mutation refers to a mutation in amino acid 478 of the S protein of the virus in which there is a change from threonine (T) to lysine (K). The " P681R" mutation refers to a mutation in amino acid 681 of the S protein of the virus in which there is a change from proline (P) to arginine (R).

The molecular beacons which comprise, preferably consist of, SEQ ID NO: 6-7, used in the method of the invention allows the detection of SARS-CoV-2 or any of its variants, preferably the strains B.1.1.7, B.1.351, P.1 and/or B.1.617.2. This means that the same molecular beacon can be used to detect different variants of SARS-CoV-2.

In another preferred embodiment, the RNA regions selected of the SARS-CoV-2 are Spike and/or Orf8 proteins. The molecular beacon which comprises, preferably consists of, the SEQ ID NO: 6 comprises an oligo complementary to the Spike protein of SARS-CoV-2 and the molecular beacon which comprises, preferably consists of, the SEQ ID NO: 7 comprises an oligo complementary to the Orf8 protein of SARS-CoV-2.

The molecular beacons are labelled with different fluorophores, preferably FAM, HEX, TET, JOE, VIC, Cy3, NED, TAMRA, Cy3.5, ROC, Texas Red or Cy5, more preferably FAM or HEX.

As mentioned above, the same molecular beacon can be used for the detection of different variants of SARS-CoV-2. However, specific beacons can be designed for the detection of these variants. Therefore, in a particular embodiment of the method of the invention, the molecular beacon which is designed for the detection of the strain B.1.1.7. comprises, preferably consists of, SEQ ID NO: 46 (5'-GTAACCAAGACCATAAGTGGGTTACGTCATCCGACGTCGGATGAC-3') and the molecular beacon designed for the detection of the WT strain comprises, preferably consists of, SEQ ID NO: 47 (5'-GTAACCAAGACCATTAGTGGGTTACGTCATCCGACGTCGGATGAC-3').

The method of the invention is based on the detection of single-stranded nucleic acids, preferably single-stranded viruses, more preferably ssRNA viruses, in a biological sample isolated from a subject. The means used for this detection of single-stranded nucleic acids can be part of a kit.

Thus, a second aspect of the present invention relates to a kit, hereinafter "the kit of the invention", comprising means for detecting *in vitro* the presence of at least one single- stranded nucleic acid, preferably a ssDNA or a ssRNA virus, more preferably a ssRNA virus, in an isolated sample for a subject.

The terms "detect" or "detecting", *"in vitro"* and "subject" have been defined or explained above, and these definitions are applicable to the kit of the invention.

The kit of the invention comprises, at least:

    i) a FokI endonuclease; and
    ii) at least one molecular beacon, wherein said beacon comprises:

        a) a double-stranded DNA fragment comprising a FokI recognition site;
        b) an oligonucleotide complementary to a nucleic acid region of the single-stranded nucleic acid;
        c) a fluorophore at one of the ends, preferably at the 5' end; and
        d) a fluorescence quencher molecule at the end opposite to the end of (c), preferably at the 3' end.

In addition to these means, the kit of the invention can comprise other components useful in the implementation of the present invention, such as, buffers, material supports, positive and/or negative control components, etc. In addition to the aforementioned components, the kit can also include instructions for practicing the object of the invention. These instructions may be present in the aforementioned kit in a variety of forms, one or more of which may be present in the kit. One form in which these instructions may be present is as printed information on a suitable medium or substrate, e.g., a sheet or sheets of paper on which the information is printed, on the kit packaging, on a package insert, etc. Another medium would be a computer readable medium, e.g., CD, USB, etc., on which the information has been recorded. Another medium that may be present is a website address that can be used via the Internet to access the information at a remote site. Any convenient medium may be present in the kit.

In a particular embodiment, the kit of the invention further comprises means for amplifying the nucleic acid target, preferably by rolling circle amplification (RCA), wherein said means comprises: (i) at least one padlock probe, (ii) a DNA ligase and (iii) a Phi29 DNA polymerase or a form of the Phi29 DNA polymerase. The kit may comprise other useful components for the amplification, as specific primers or probes, dNTPs, and the universal rolling circle primer (SEQ ID NO: 45, Table 2).

The terms "padlock probe", "DNA ligase", "primer", "dNTP" and "Phi29 DNA polymerase" have been defined or explained above, and these definitions are applicable to the kit of the invention.

In another particular embodiment, the fluorophore of the molecular beacon, which is preferably at the 5' end of the hairpin, is FAM, HEX, TET, JOE, VIC, Cy3, NED, TAMRA, Cy3.5, ROC, Texas Red or Cy5, preferably FAM or HEX.

In another particular embodiment, the quencher of the molecular beacon, which is preferably at the 3' end of the hairpin, is BHQ1 or BHQ2, preferably BHQ1.

In a preferred embodiment, the fluorophore-quencher pair of the molecular beacon is selected from the list: FAM-BHQ1, HEX-BHQ1, TET-BHQ1, JOE-BHQ1, VIC- BHQ1, Cy3-BHQ1, Cy3-BHQ2, NED-BHQ1, NED-BHQ2, TAMRA-BHQ2, Cy3.5- BHQ2, ROX-BHQ2, Texas Red-BHQ2 or Cy5-BHQ2. In a more preferred embodiment, the fluorophore-quencher pair of the molecular beacon is FAM-BHQ1 or HEX-BHQ1.

As mentioned above, the single-stranded nucleic acid is, preferably, a single-stranded DNA (ssDNA) virus or a single-stranded RNA (ssRNA) virus, more preferably a ssRNA virus. Thus, in another particular embodiment, the ssRNA virus is one that causes nasal and/or oral infections or has an oral and/or nasal presence in the subject.

In another particular embodiment, the single-stranded RNA virus is selected from the list consisting of: *Influenza A virus, Influenza B virus, Influenza C virus, Alphacoronavirus, Betacoronavirus,* SARS-CoV-2, *Rhinovirus, Parechovirus,* Parainfluenza and *Pneumovirus.*

The term "sample" has been defined or explained above, and this definition is applicable to the kit of the invention. In a preferred embodiment, the sample is a biological sample. The term "biological sample" has been defined or explained above, and this definition is applicable to the kit of the invention.

In a more preferred embodiment, the biological sample is isolated from the oral or respiratory tract of the subject, most preferably, by means of a nasopharyngeal swab.

The sample is isolated from a subject. The term "subject" has been defined or explained above, and this definition is applicable to the kit of the invention.

In another particular embodiment, the kit of the invention further comprises means to detect, in the isolated sample, the presence of more than one single-stranded nucleic acid, or more than one region of the same single-stranded nucleic acid, preferably a ssDNA virus or a ssRNA virus, more preferably a ssRNA virus. These means comprise:

a) more than one molecular beacon, each comprising a different oligonucleotide complementary to a different region of the same single-stranded nucleic acid, or a different oligonucleotide complementary to a different single-stranded nucleic acid; and
b) more than one padlock probe.

In a preferred embodiment, the kit of the invention comprises means to detect, in the isolated sample, the presence of more than one ssRNA virus, or more than one region of the same ssRNA virus. These means comprise:

a) more than one molecular beacon, each comprising a different oligonucleotide complementary to a different RNA region of the same ssRNA virus, or a different oligonucleotide complementary to a different ssRNA virus; and
b) more than one padlock probe.

The kit of the invention may comprise other components useful to detect the presence of more than one ssRNA virus or more than one RNA region of the same ssRNA virus in the isolated sample, such as specific primers and probes. The terms "primer", "probe" and "padlock probe" have been defined or explained above, and these definitions are applicable to the kit of the invention.

The usefulness of the kit of the invention and of the method of the invention for the detection of at least one single-stranded nucleic acid, preferably a ssDNA or a ssRNA virus, more preferably a ssRNA virus, in an isolated sample from a subject has been shown in the preceding paragraphs. Thus, a third aspect of the invention is the use of the kit of the invention in the method of the invention.

## DESCRIPTION OF THE DRAWINGS

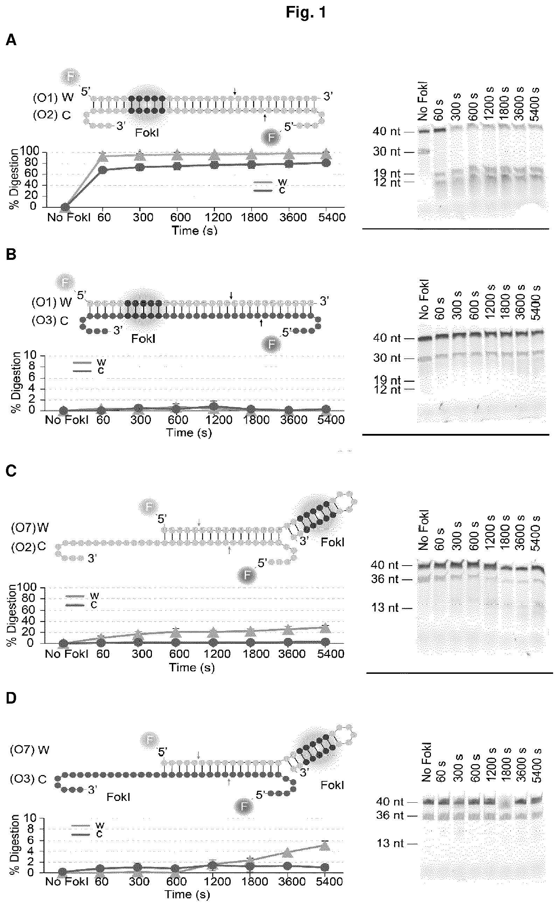

**Fig. 1. FokI-assisted digestion of DNA duplexes and DNA: RNA hybrids. (A)** Pictorgram of DNA duplexes and **(B)** DNA: RNA hybrids indicating the 5' end labelling of the oligonucleotides with Cy5.5 (dark grey) or IRD800 (light grey). W and C represent the conventional positional nomenclature for the different molecules in the duplex or heteroduplex substrate (W = Watson strand, C = Crick strand), while light grey of dark grey dots denote the type of nucleic acid (DNA or RNA, respectively). Denaturing PAGE RNA gels (right panel) show the restriction products obtained upon incubation of FokI and the corresponding substrate at different time points, measured in seconds. The left axis indicates the size (number of nucleotides) of the corresponding oligonucleotides or digested products. **(C)** and **(D)** Same as A and B, but the schema depicts the pictogram and the structural hybridization properties of the hairpin oligonucleotide probes with a complementary DNA strand **(C)** or RNA strand **(D)**. For all panels, data represent mean $\pm$ standard deviation of each of the conditions.

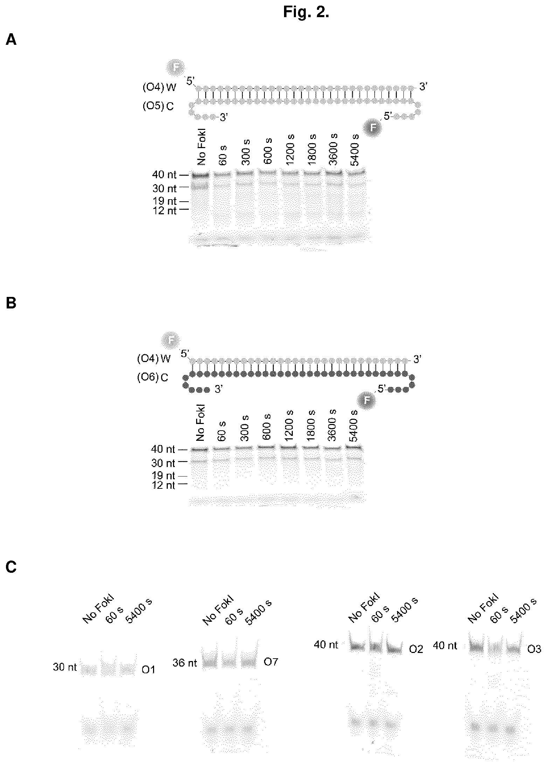

**Fig. 2. No STAR activity is observed upon FokI digestion of DNA duplexes and DNA: RNA hybrids. (A)** Pictogram of DNA duplexes of **(B)** DNA: RNA hybrids that lacked a FokI restriction site illustrating the 5' end labelling of the oligonucleotides with Cy5.5 (dark grey) or IRD800 (light grey). W and C represent the conventional positional nomenclature for the different molecules in the duplex or heteroduplex substrate (W = Watson strand, C = Crick strand), while light grey of dark grey dots denote the type of nucleic acid (DNA or RNA, respectively). Denaturing PAGE RNA gels show the restriction products obtained upon incubation of FokI and the corresponding substrate at different time points. The left axis indicates the expected size (number of nucleotides) of the corresponding RE products. **(C)** Denaturing PAGE gels show the restriction products obtained upon incubation of FokI and each of the different individual substrates for 90 min at 37ₒC. The size and the code of the corresponding DNA or RNA oligonucleotides is indicated. The legend shows the experimental combinations used in the restriction assay and measurements indicate the percentage digestion of the probes at the end of the reaction.

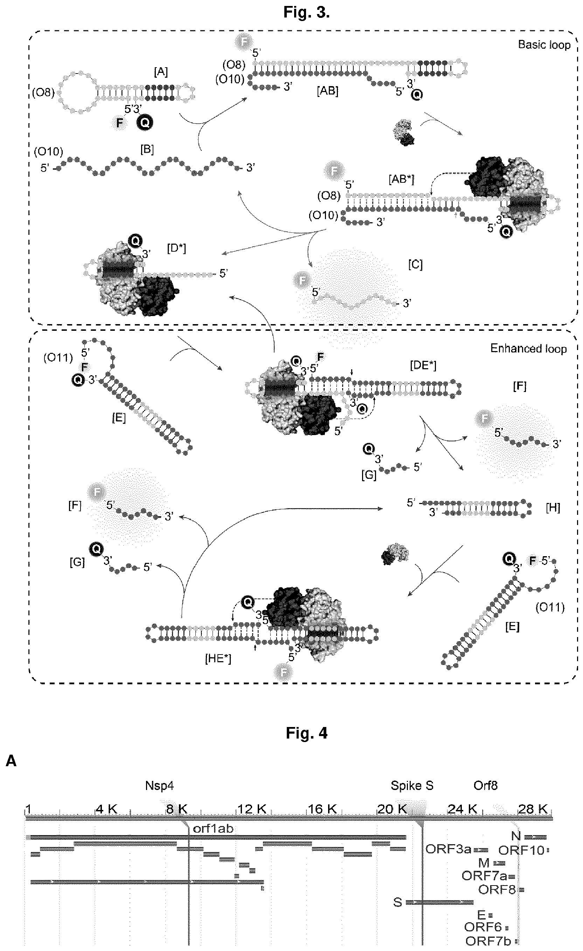

**Fig. 3. Working principle of FokI-assisted digestion of nucleic acids.** Schema depicting the different stages of the signal amplification reaction. In the presence of a target molecule, the custom oligonucleotide triggers the hybridization step. Depending on the number of components present in the reaction, a cascade of different feedback loops may be activated, resulting in the enhancement of the fluorescent signal of the assay. Dashed circles indicate the molecular steps required for the basic (at the top) or enhanced (at the bottom) signal amplification reaction.

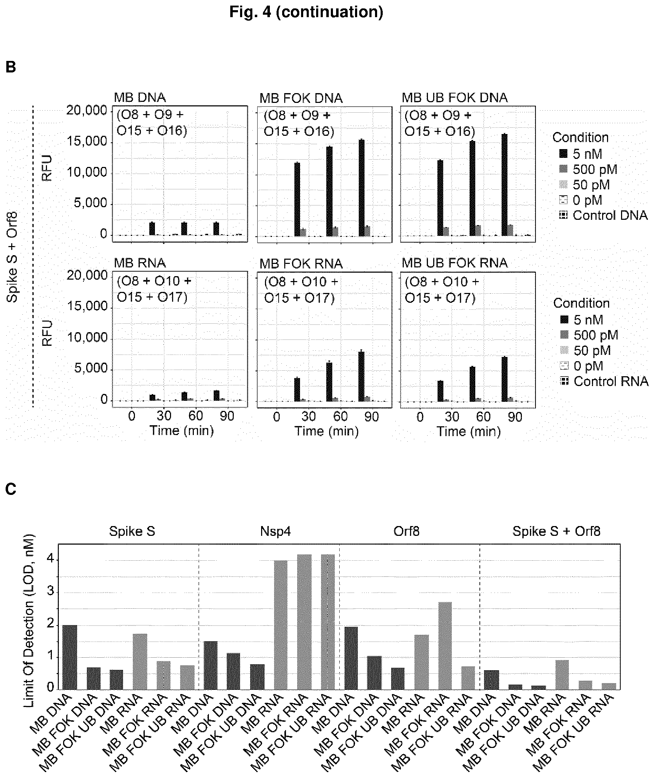

**Fig. 4. Enhanced detection of SARS-CoV-2 sequences via FokI-assisted digestion of DNA: RNA hybrids. (A)** Graphical representation of the SARS-CoV-2 genome. Open reading frames, as well as peptides generated from the long ORF1a/b are represented by dark boxes. The location of the custom oligonucleotides and the synthetic DNA and RNA probes corresponding to the Nsp4, Spike and Orf8 regions is indicated by vertical lines. **(B)** Bar plots displaying the fluorescence detection measurements of a conventional hybridization assay (left panel), a basic FokI-assisted signal amplification assay (middle panel) and an extended FokI-assisted signal amplification assay in the presence of universal hairpin oligonucleotides (right panel). **(C)** Bar plot represents the limit of detection (nm range) for the different conditions (DNA or RNA) and sequence combinations as calculated from the experimental data.

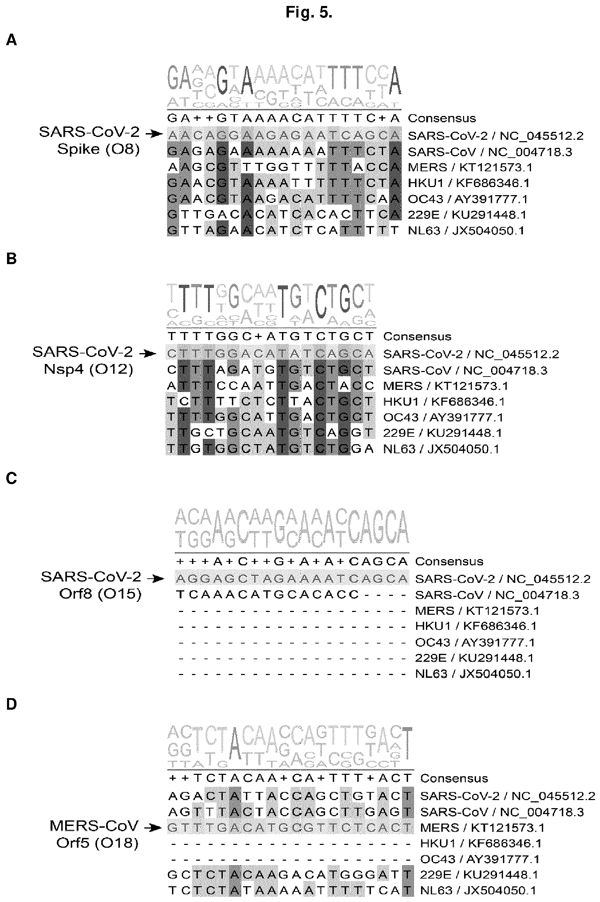

**Fig. 5. Design of target DNA/RNA sequences and reporter custom oligonucleotides. (A)** Multiple Sequence Alignment (MSA) including SARS-CoV-2 sequences targeted by the hairpin oligonucleotide against Spike, **(B)** Nsp4, **(C)** Orf8 loci and the sequences of 6 other human Beta coronaviruses at the abovementioned genomic regions. **(D)** The graph represents the MSA sequences targeted by the hairpin oligonucleotide against the Orf5 region of MERS.

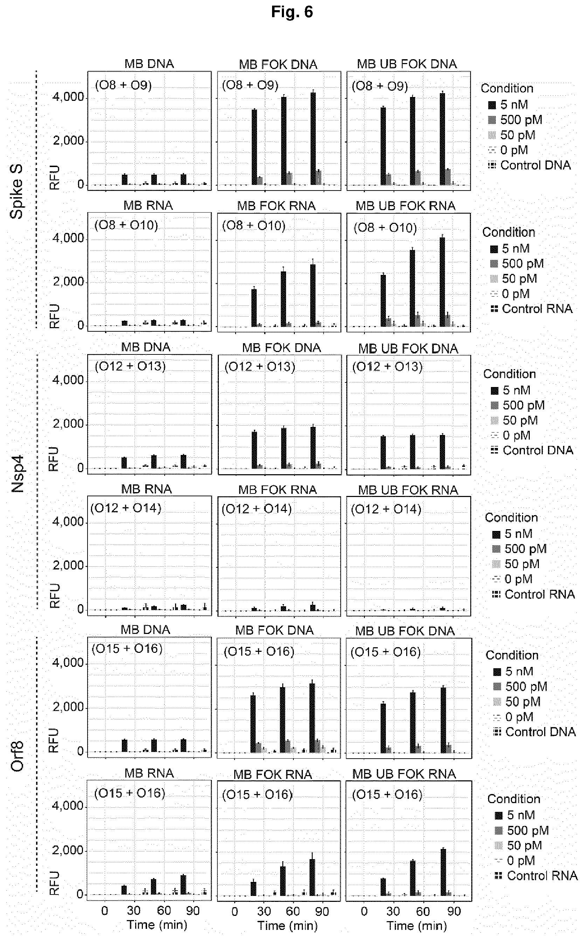

**Fig. 6. Enhanced detection of individual SARS-CoV-2 sequences.** Bar plots showing the fluorescence detection for the case of the custom oligonucleotides designed against Spike (upper graph), Nsp4 (middle graph) and Orf8 (lower graph) regions. Different concentrations of DNA (upper panels) or RNA (lower panels) were used.

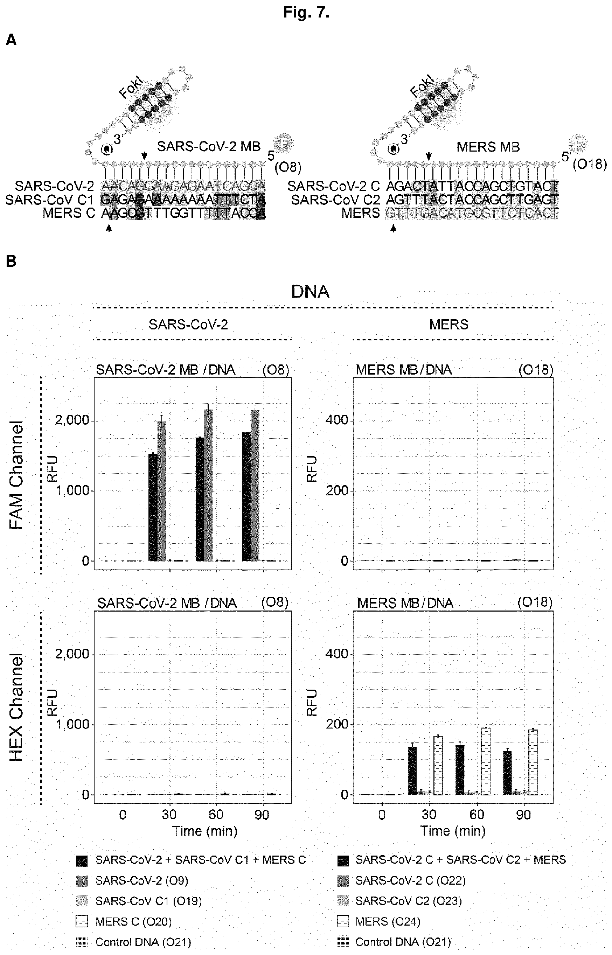

**Fig. 7. Specificity of the FokI-assisted signal amplification reaction in the context of DNA duplexes. (A)** Schema depicting the DNA sequence and the multiple sequence alignment of the human Beta coronaviruses SARS-CoV-2, SARS-CoV and MERS interrogated in these experiments. The structure and the hybridization pattern of the different custom oligonucleotides is also indicated. **(B)** Bar plots illustrating the fluorescence detection measurements of the basic FokI-assisted signal amplification assay simultaneously recorded in the FAM and the HEX channels. The different DNA substrate combinations are indicated in the bottom legend.

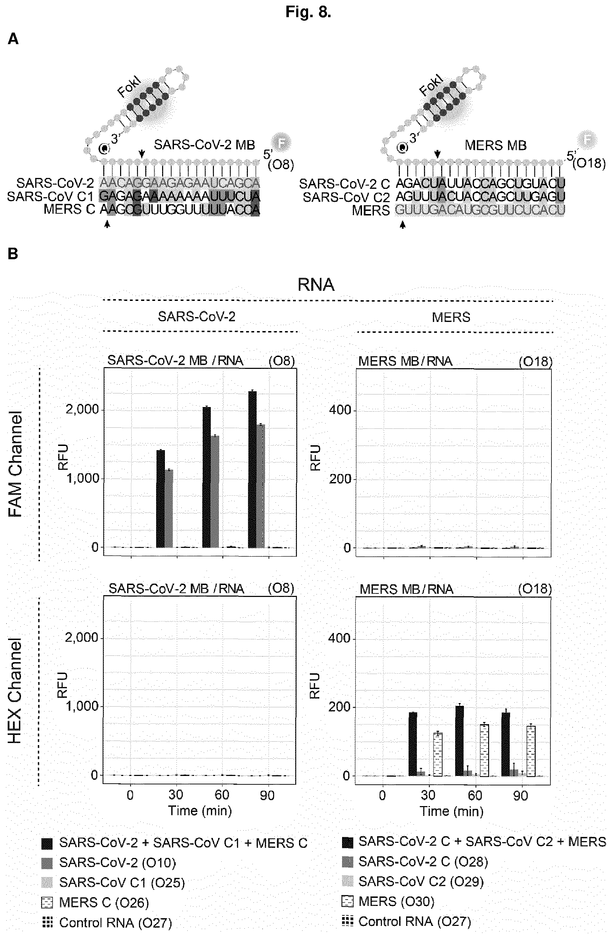

**Fig. 8. Specificity of the FokI-assisted signal amplification reaction in the context of DNA: RNA hybrids. (A)** Schema depicting the RNA sequence and the multiple sequence alignment of the human Beta coronaviruses SARS-CoV-2, SARS-CoV and MERS interrogated in these experiments. The structure and the hybridization pattern of the different custom oligonucleotides is also indicated. **(B)** Bar plots illustrating the fluorescence detection measurements of the basic FokI-assisted signal amplification assay simultaneously recorded in the FAM and the HEX channels. The different RNA substrate combinations are indicated in the bottom legend.

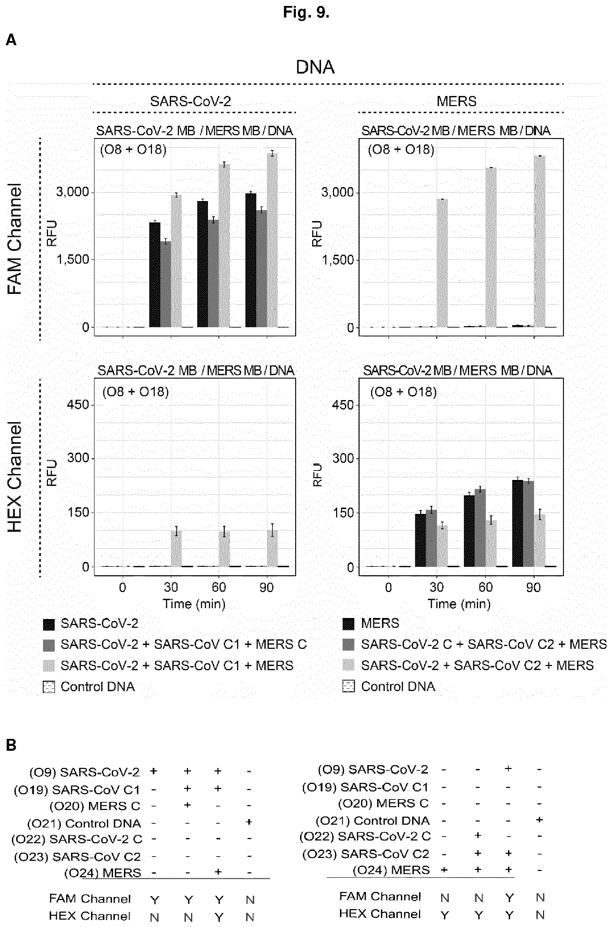

**Fig. 9. FokI-assisted signal amplification allows the simultaneous detection of multiple DNA duplexes. (A)** Bar plots indicating the fluorescence detection measurements of the basic FokI-assisted signal amplification assay simultaneously recorded in the FAM and the HEX channels and in the presence of both custom oligonucleotides. The different DNA substrate combinations are shown in the legend **(B)** The schema summarizes the detection results obtained with the combination of DNA substrates and the combination of reporter oligonucleotides in each of the detection channels.

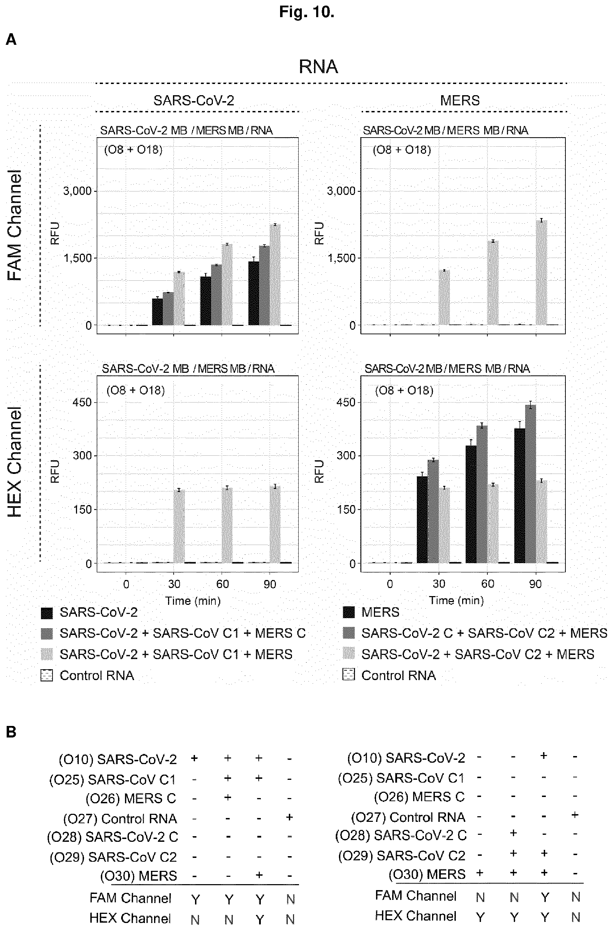

**Fig. 10. FokI-assisted signal amplification allows the simultaneous detection of multiple DNA: RNA hybrids. (A)** Bar plots indicating the fluorescence detection measurements of the basic FokI-assisted signal amplification assay simultaneously recorded in the FAM and the HEX channels and in the presence of both custom oligonucleotides. The different RNA substrate combinations are shown in the legend **(B)** The schema summarizes the detection results obtained with the combination of RNA substrates and the combination of reporter oligonucleotides in each of the detection channels.

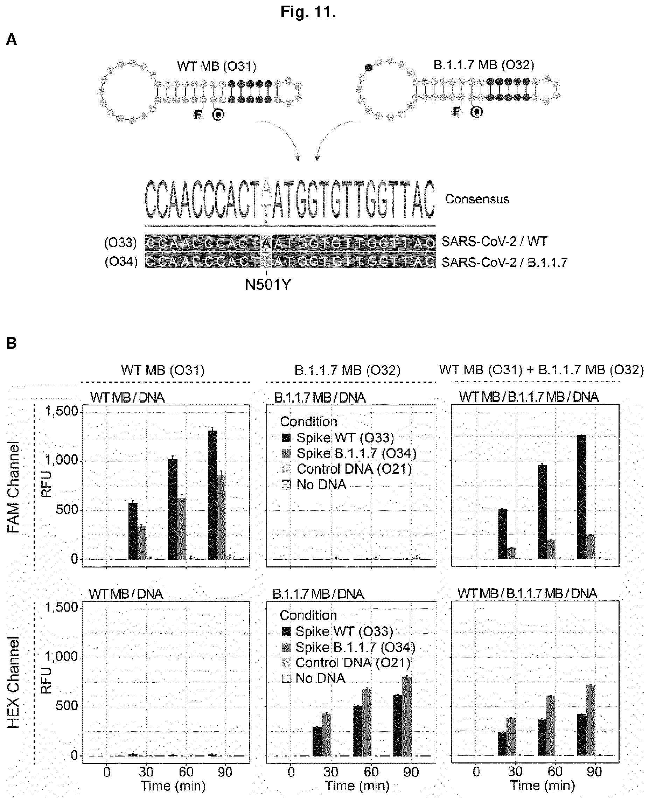

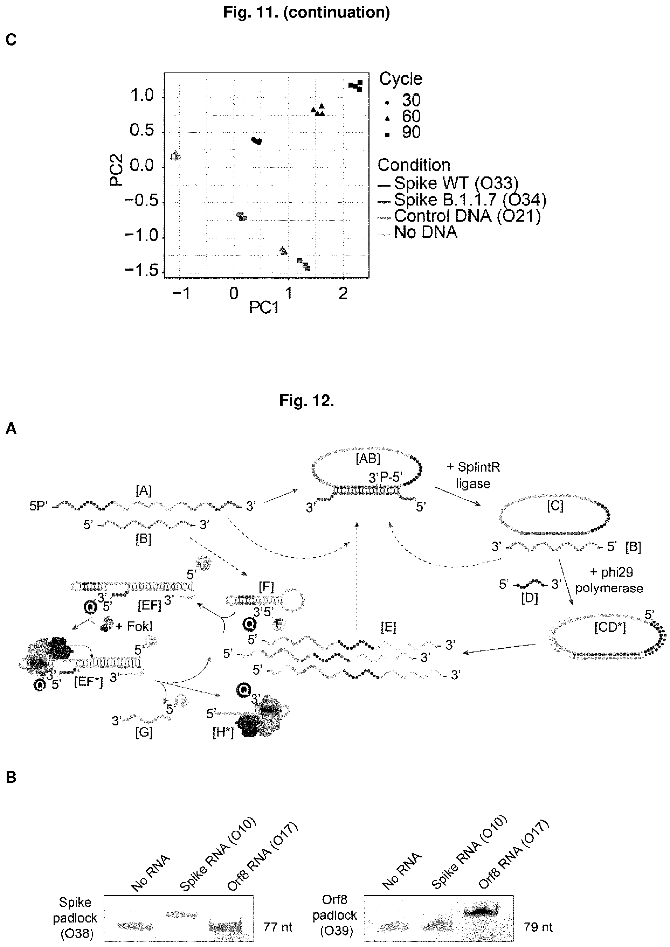

**Fig. 11. FokI-assisted signal amplification discriminates between known SARS-CoV-2 variants. (A)** Schema reflects the DNA sequence of the reporter custom structures (WT-MB = 6-FAM, B.1.1.7 MB = HEX, Q = BHQ1 quencher) and the sequence alignment of the wild type and the B.1.1.7 SARS-CoV-2 variants in the vicinity of the interrogated N501Y position. **(B)** Bar plots indicating the fluorescence detection measurements of the basic FokI-assisted signal amplification assay simultaneously recorded in the FAM and the HEX channels and in the presence of WT, B.1.1.7 or both reporter custom oligonucleotides. **(C)** Principal component analysis of the conditions in reactions containing both reporter custom oligonucleotides reflecting differential signal trajectories for the different SARS-CoV-2 variants at the indicated time points. Combinations of dot grey tone and shape represent different substrates and incubation times, respectively.

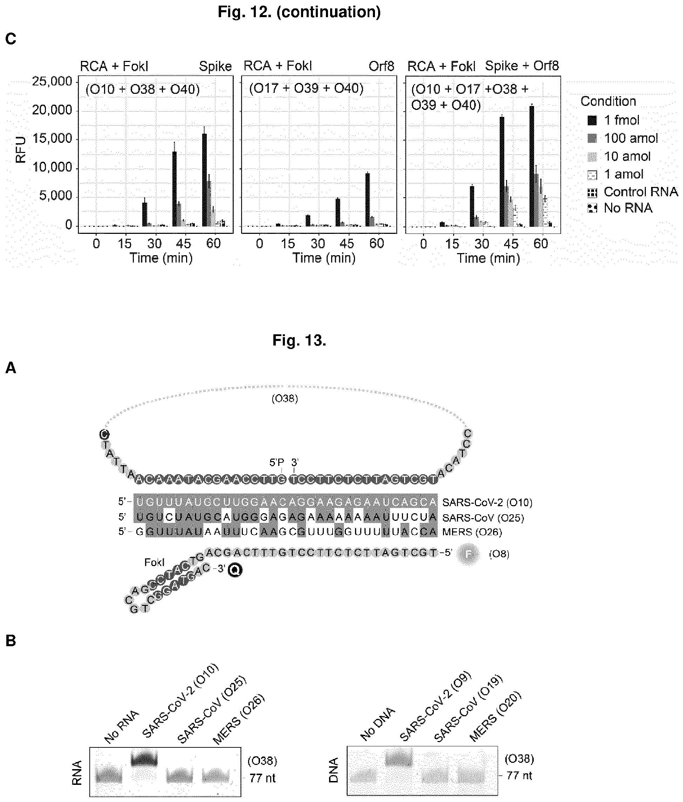

**Fig. 12. Molecular coupling between FokI-assisted signal amplification and RCA. (A)** Schema depicting the different stages of the RCA reaction and the molecular coupling with the FokI-assisted signal amplification method. Dashed lines indicate potential interactions or feedback loops that may co-exist between the reaction steps indicated. **(B)** Graphs indicate the results of the ligation assay including Spike or Orf8 padlock probes and their complementary target RNAs. The size of the linear padlock probe is indicated. **(C)** Bar plots illustrating the fluorescence detection measurements of an RCA reaction coupled with the FokI-assisted signal amplification system in the presence of custom oligonucleotides against Spike (left panel), Orf8a (middle panel) or both reporter constructs (right panel). The different target concentrations used in this experiment are indicated in the legend on the right.

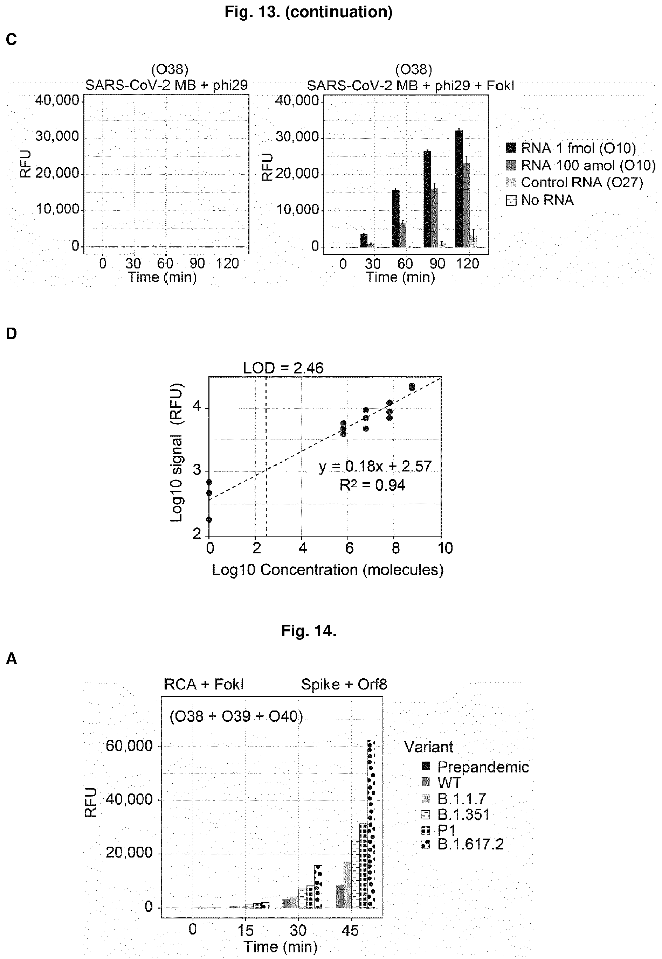

**Fig. 13. Specificity and Limit of Detection of the RCA enhanced reaction in the context of FokI-assisted signal amplification assay. (A)** Schema depicting the structure of a specific padlock probe (upper oligonucleotide) or custom oligonucleotides (lower sequence) upon hybridization with its cognate target SARS-CoV-2 Spike RNA. Middle sequences represent the results of a MSA against the Spike SARS-CoV-2 region. **(B)** Graphs illustrate the results of the ligation assay including padlock probes against the SARS-CoV-2 Spike region and their related complementary target RNAs (left image) or DNAs (right image), or oligonucleotides corresponding to orthologous regions of other SARS-like coronaviruses (SARS-CoV or MERS). **(C)** Bar plots indicate the fluorescence detection measurements of a RCA reaction coupled with the FokI-assisted signal amplification system in the presence of custom oligonucleotides against the Spike region of SARS-CoV-2 in the absence (left panel), or in the presence (right panel) of 5 units of FokI RE. **(D)** Scatter plot depicting the linear relationship between increasing concentrations of RNA target (x-axis, Spike region, 1 amol, 10 amol, 100 amol, 1 fmol and unrelated control RNA), and the intensity of the fluorescent measurements obtained from the molecular coupling between RCA and the FokI-assisted signal amplification system (y-axis). Both relative fluorescence units and the number of target molecules included in the assay are represented in Log10 scale.

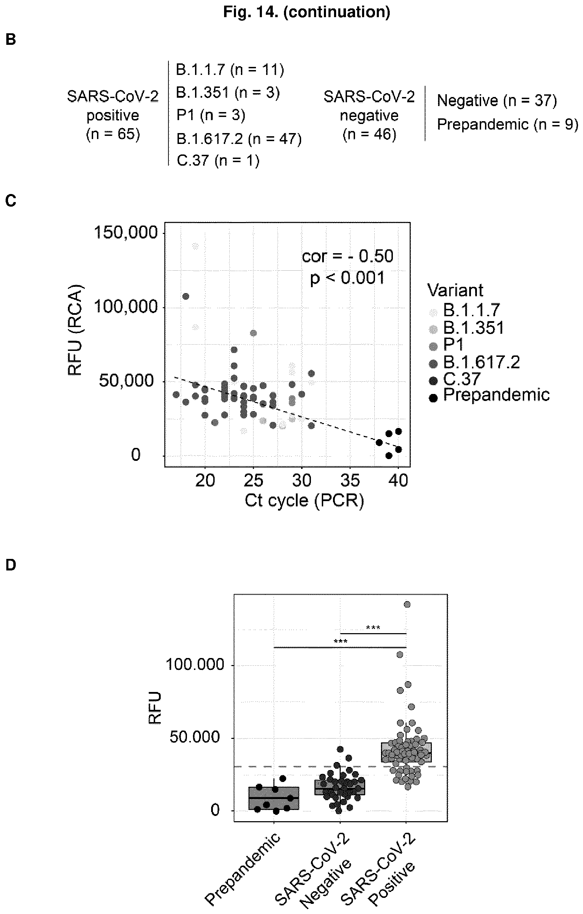

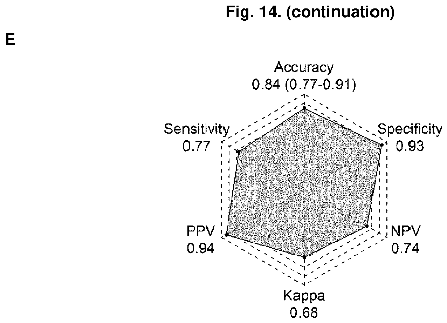

**Fig. 14. Detection of SARS-CoV-2 in samples from COVID-19 patients. (A)** Bar plots showing the fluorescence detection measurements of an RCA reaction coupled with the FokI-assisted signal amplification system in the presence of custom oligonucleotides against the Spike and Orf8 regions of SARS-CoV-2. Different conditions corresponding to the different SARS-CoV-2 strains are indicated in the legend on the right. **(B)** Schema indicates the number of SARS-CoV-2 positive, or control cases used for validation purposes. The number of samples corresponding to each validated strain is also indicated. **(C)** Scatter plot reflecting the relationship between RT-qPCR (x-axis) and FokI-assisted RCA results in the samples. For RT-qPCR results, the observed Ct is indicated. **(D)** Box plots showing the relative fluorescence units of SARS-CoV-2 positive or control cases as obtained from the previous RCA coupled FokI-assisted signal amplification assays. Dots correspond to individual samples and dashed line indicates the relative fluorescence corresponding to the z-score cut-off used for statistical purposes (*** = p-value < 0.001, one-tailed Welch's T-test). **(E)** Radar plots illustrate the metrics and performance of the FokI-assisted signal amplification method as compared to the gold standard qRT-PCR approach.

**Examples**

Having now generally described the invention, the same will be more readily understood through reference to the following examples, which are provided by way of illustration and are not intended to be limiting of the present invention.

**1. Material al methods**

**1.1. Strand-specific analysis of FokI endonuclease activity**

Various combinations of synthetic RNA and DNA sequences including FokI restriction sites were designed and labelled with either IRD800 or Cy5.5 fluorophores at their 5' end. All oligonucleotides used in this study (Table 3) were synthetized by Metabion (Metabion International AG, BY,Germany). Prior to the endonuclease reaction, annealing reactions included a DNA duplex or DNA: RNA heteroduplex mixture at a concentration of 1 $\mu$M in a final volume of 100 $\mu$l of RNAse-free water. Samples were denatured at 95ºC for 2 minutes and left at room temperature for 5 minutes to achieve full annealing between complementary oligonucleotides. Restriction enzyme digestions were performed at 37ºC for 1, 5, 10, 20, 30, 60 and 90 minutes in a reaction mix comprising 2 $\mu$l of NEB 10X Cutsmart buffer (New England Biolabs, MA, USA. 1$\times$ Buffer composition: 50 mM Potassium Acetate, 20 mM Tris-Acetate, 10 mM Magnesium Acetate, 100 $\mu$g/ml BSA, pH 7.9 at 25ºC), 50nM (1 pmol) of the duplex or heteroduplex substrate and 5 units of the RE FokI (New England Biolabs, MA, USA) in a final volume of 20 $\mu$l. All conditions were tested at least in duplicate in two independent experiments. Reaction products were mixed with one volume of 2X denaturing gel-loading urea buffer (90 mM Tris, 90 mM Boric acid, 2 mM EDTA, pH 8, 12% glycerol, 0.01% Bromophenol blue, 7 M Urea) and heated to 95ºC for 2 minutes. 20 $\mu$l aliquots were resolved by denaturing polyacrylamide gel electrophoresis (PAGE) in 15% polyacrylamide/urea/TBE denaturing pre-cast gels (BioRad, CA, USA). IRD800 and Cy5.5 fluorescence corresponding to the different oligonucleotides was monitored by gel scanning using a LI-COR Fc Odyssey variable mode imager (LI-COR biosciences, NE, USA). Excitation wavelengths of 685nm (with 700nm channel) and 785nm (with 800nm channel) were used for Cy5.5 and IRD800 scans, respectively. Densitometric analyses of the corresponding bands were performed with ImageStudio software (LI-COR biosciences, NE, USA). To facilitate the quantification of the digested products, a prior conversion of the 700 nm or 800 nm channels into grayscale images was performed.

**Table 3.** List of primers that have been used in the examples

| Name | Code | 5'Modification | 3'Modification | Sequence |
|---|---|---|---|---|
| DNA 1 Watson | O1 | IRD800 | - | SEQ ID NO: CAGTCGGATGACATGGGTACCA 48 TCGTCATC |
| DNA 1 Crick | O2 | Cy5.5 | - | SEQ ID NO: CTAGAGATGACGATGGTACCCA 49 TGTCATCCGACTGCCTAC |
| RNA 1 Crick | O3 | Cy5.5 | - | SEQ ID NO: CUAGAGAUGACGAUGGUACCC 50 AUGUCAUCCGACUGCCUAC |
| DNA 2 Watson | O4 | IRD800 | - | SEQ ID NO: GTCAGCCTACTGTACCCATGGT 51 AGCAGTAG |
| DNA 2 Crick | O5 | Cy5.5 | - | SEQ ID NO: GATCTCTACTGCTACCATGGGT 52 ACAGTAGGCTGACGGATG |
| RNA 2 Crick | O6 | Cy5.5 | - | SEQ ID NO: GAUCUCUACUGCUACCAUGGG 53 UACAGUAGGCUGACGGAUG |

(continued)

| Name | Code | 5'Modification | 3'Modification | Sequence |
|------|------|----------------|----------------|----------|
| DNA3 Watson | O7 | IRD800 | - | SEQ ID NO: 54 TGGGTACCATCGTCATGTCATCCGACGTCGGATGAC |
| MB1 SARSCOV2 (Spike) | O8 | FAM | BHQ1 | SEQ ID NO: 6 TGCTGATTCTCTTCCTGTTTCAGCAGTCATCCGACGTCGGATGAC |
| DNA1 SARSCOV2 (Spike) | O9 | - | - | SEQ ID NO: 17 CTTGGAACAGGAAGAGAATCAGCAACTGTG |
| RNA1 SARSCOV2 (Spike) | O10 | - | - | SEQ ID NO: 28 CUUGGAACAGGAAGAGAAUCAGCAACUGUG |
| Fuel MB1 | O11 | FAM | BHQ1 | SEQ ID NO: 55 TGCTGAGCAGCCTCCATCCACTCTGCGCAGAGTGGATGGAGGCTGC |
| MB2 SARSCOV2 (Nsp4) | O12 | FAM | BHQ1 | SEQ ID NO: 56 TGCTGATATGTCCAAAGTCAGCAGTCATCCGACGTCGGATGAC |
| DNA2 SARSCOV2 (Nsp4) | O13 | - | - | SEQ ID NO: 57 ATTGGTGCTTTGGACATATCAGCATCTATA |
| RNA2 SARSCOV2 (Nsp4) | O14 | - | - | SEQ ID NO: 58 AUUGGUGCUUUGGACAUAUCAGCAUCUAUA |
| MB3 SARSCOV2 (Orf8) | O15 | FAM | BHQ1 | SEQ ID NO: 7 TGCTGATTTTCTAGCTCCTTCAGCAGTCATCCGACGTCGGATGAC |
| DNA3 SARSCOV2 (Orf8) | O16 | - | - | SEQ ID NO: 18 AGAGTAGGAGCTAGAAAATCAGCACCTTTA |
| RNA3 SARSCOV2 (Orf8) | O17 | - | - | SEQ ID NO: 29 AGAGUAGGAGCUAGAAAAUCAGCACCUUUA |
| MB1 MERS (Orf5) | O18 | HEX | BHQ1 | SEQ ID NO: 59 AGTGAGAACGCATGTCAAACCTCACTGTCATCCGACGTCGGATGAC |

(continued)

| Name | Code | 5'Modification | 3'Modification | Sequence |
|---|---|---|---|---|
| Control1 DNA SARSCOV | O19 | - | - | SEQ ID NO: 60 CATGGGAGAGAAAAAAATTTCTAATTGTG |
| Control1 DNA MERS | O20 | - | - | SEQ ID NO: 61 ATTTCAAGCGTTTGGTTTTTACCAATTGCA |
| Control DNA | O21 | - | - | SEQ ID NO: 62 TGCTAGAAAACCGCGTTTCTACGACTGGTG |
| Control1 DNA SARSCOV2 | O22 | - | - | SEQ ID NO: 63 ACTTCAGACTATTACCAGCTGTACTCAACT |
| Control2 DNA SARSCOV | O23 | - | - | SEQ ID NO: 64 ACCGAAGTTTACTACCAGCTTGAGTCTACA |
| DNA4 MERS (Orf5) | O24 | - | - | SEQ ID NO: 65 CCACTGTTTGACATGCGTTCTCACTTTATT |
| Control1 RNA SARSCOV | O25 | - | - | SEQ ID NO: 66 CAUGGGAGAGAAAAAAAUUUCUAAUUGUG |
| Control1 RNA MERS | O26 | - | - | SEQ ID NO: 67 AUUUCAAGCGUUUGGUUUUUACCAAUUGCA |
| Control RNA | O27 | - | - | SEQ ID NO: 68 UGCUAGAAAACCGCGUUUCUACGACUGGUG |
| Control2 RNA SARSCOV2 | O28 | - | - | SEQ ID NO: 69 ACUUCAGACUAUUACCAGCUGUACUCAACU |
| Control2 RNA SARSCOV | O29 | - | - | SEQ ID NO: 70 ACCGAAGUUUACUACCAGCUUGAGUCUACA |
| RNA4 MERS (Orf5) | O30 | - | - | SEQ ID NO: 71 CCACUGUUUGACAUGCGUUCUCACUUUAUU |

(continued)

| Name | Code | 5'Modification | 3'Modification | Sequence |
|------|------|----------------|----------------|----------|
| MB WT (Spike) | O31 | FAM | BHQ1 | SEQ ID NO: 47 GTAACCAAGACCATTAGTGGGTTACGTCATCCGACGTCGGATGAC |
| MB B.1.1.7 (Spike) | O32 | HEX | BHQ1 | SEQ ID NO: 46 GTAACCAAGACCATAAGTGGGTTACGTCATCCGACGTCGGATGAC |
| RNA SC2 Wt | O33 | - | - | SEQ ID NO: 72 CCAACCCACTAATGGTGTTGGTTAC |
| RNA SC2 Mut | O34 | - | - | SEQ ID NO: 73 CCAACCCACTTATGGTGTTGGTTAC |
| MB1 SARSCOV2 (Spike) 11% 2OMe | O35 | FAM | BHQ1 | SEQ ID NO: 74 TGCTGATU*C*TCTTCCTGTTTCAG*C*AGTCATCCGAC*GTCGGATGAC |
| MB1 SARSCOV2 (Spike) 22% 2OMe | O36 | FAM | BHQ1 | SEQ ID NO: 75 TGC*TGAU*TC*TCU*TCCTGTU*TCAG*CAG*TCATCCGA*CGU*CGGATGAC |
| MB1 SARSCOV2 (Spike) 62% 2OMe | O37 | FAM | BHQ1 | SEQ ID NO: 76 U*G*C*U*G*A*U*U*C*U*C*U*TCCTGTTU*C*A*G*C*A*G*U*CATCCG*A*C*G*U*C*GGATGA*C* |
| Spike padlock RCA | O38 | P | - | SEQ ID NO: 39 GTTCCAAGCATAAACAATTATCCCTATAGTGAGTCGTATTAGAATTTTCCCGGATCCTACATGCTGATTCTCTTCCT |
| Orfa8a padlock RCA | O39 | P | - | SEQ ID NO: 40 CCTACTCTAATATACCATTATCCCTATAGTGAGTCGTATTAGAATTTTCCCAGTCGTACGCATGCTGATTTTCTAGCT |
| Universal RCA primer | O40 | - | - | SEQ ID NO: 45 AATTCTAATACGACTCACTATAGG^G^ |

(continued)

| Name | Code | 5'Modification | 3'Modification | Sequence |
|---|---|---|---|---|
| 2019-nCoV_N1-F | O41 | - | - | SEQ ID NO: 77 GACCCCAAAATCAGCGAAT |
| 2019-nCoV_N1-R | O42 | - | - | SEQ ID NO: 78 TCTGGTTACTGCCAGTTGAATCTG |
| 2019-nCoV_N1-P-VIC | O43 | VIC | 3IABkFQ | SEQ ID NO: 79 ACCCCGCAT/ZEN/TACGTTTGGTGGACC |

* = 2'-O-Methyl RNA modification
^ = 3' - Phosphorothioate modification

The ZEN™ quencher is a versatile modification originally developed by IDT as an internal quencher for qPCR 5'-nuclease assay fluorescence-quenched probes. This quencher is placed internally between the 9th and 10th base from the reporter dye on the 5' end of a probe sequence. A traditional probe is 20-30 bases in length with a terminal dye and quencher. The internal ZEN quencher thus shortens the distance between dye and quencher, and in combina-tion with the terminal 3' quencher, provides a higher degree of quenching and lowers initial background.

## 1.2. Design of synthetic oligonucleotides against human coronaviruses

The FASTA sequences corresponding to 7 human infectious coronaviruses (SARS-CoV-2, SARS-CoV, MERS, HKU1, OC43, HCoV-229E and NL63) were downloaded from NCBI (accession numbers NC_045512.2, NC_004718.3, KT121573, KF686346.1, AY391777.1, KU291448.1, JX504050.1, respectively). Multiple sequence alignment was performed with the computational tool Clustal Omega implemented in the European Bioinformatics Institute (EBI) servers using as input the aforementioned cDNA sequences. Visualization of the multiple sequence alignments was performed with the Jalview software (v2.11).

For the identification SARS-CoV-2 sequences with common hybridization regions compatible with custom-designed oligonucleotides, inventors initially scanned the occurrence of all possible combinations of 6-mer along the reverse complement SARS-CoV-2 genome with the out-of-core k-mer counter Meryl (rev2008) (Miller,J.R et al., (2008) Aggressive assembly of pyrosequencing reads with mates. Bioinformatics, 24, 2818-2824). Manual inspection of sequences surrounding the TGCTGA hexamer was used to explore regions with maximum number of mismatches between the different human Betacoronaviruses. The results of this top-down approach led to the design of custom oligonucleotides (molecular beacons) against 3 regions of the SARS-CoV-2 genome, corresponding to the open reading frames of the Spike, Nsp4 and Orf8 proteins. The aforementioned oligonucleotides were labelled with 6-FAM fluorophore and BHQ-1 quencher at their 5' and 3' ends, respectively, and these sequences were characterized by a common hairpin region including a unique FokI restriction site. For evaluation purposes, inventors designed short synthetic RNAs and DNAs corresponding to the aforementioned SARS-CoV-2 genomic regions or to a random oligonucleotide sequence (30 nt length) in order to set up the FokI-assisted signal amplification reactions. To enhance the amplification of the fluorescent signal by the introduction of feedback loops, they also designed a universal hairpin oligonucleotide capable of hybridizing with the reaction product of any of the previous RE reactions. This universal hairpin was labelled with 6-FAM fluorophore and BHQ-1 quencher at its 5' and 3' ends, respectively, and its structure reassembled the DNA machine construct proposed by Weizmann et al., 2006.

## 1.3. Conventional hybridization reactions and FokI-assisted signal amplification assays

All reactions were performed in quadruplicate in a final reaction volume of 20 $\mu$l and at a constant temperature of 37ºC. For the conventional hybridization assays, the final concentration of the reaction components was 2 $\mu$l of NEB 10X Cutsmart buffer (New England Biolabs, MA, USA), 50 nM of custom oligonucleotides for the different SARS-CoV-2 synthetic regions, and decreasing concentrations of either target DNA, target RNA (5 nM, 500 pM, 50pM, 0 M) or 50 nM of control DNA or RNA for each corresponding reaction (10-fold excess). Basic FokI-assisted signal amplification assay was performed as indicated in the conventional hybridization protocol but including the addition of 5 units of FokI RE (New England Biolabs, MA, USA). Enhanced FokI signal amplification assay was performed as indicated in the basic

FokI-assisted signal amplification assay but was carried out in the presence of 50 nM universal hairpin oligonucleotide. After this, the fluoresce intensity of the reaction mixtures was recorded over time using a StepOnePlus™ Real-Time PCR System (Applied Biosystems, CA, USA). The excitation and emission wavelengths were configured to detect the FAM channel (488 nm and 520 nm, respectively). The reactions were maintained for 90 cycles (each cycle 1 minute at 37ºC) and fluorescence measurements for the different conditions were obtained in each of the cycles. Multicomponent data were downloaded from the instrument and raw data from two independent experiments were normalized for potential batch effects using the ComBat function of the R/Bioconductor package sva. To facilitate comparisons between the different technologies, and to avoid potential confounding effects caused by the different levels of background signals, signal intensities for the different conditions at the different time points were firstly normalized to the signal of each corresponding sample at time 0 (intra-sample normalization), and then to the signal of the non-template control (inter-sample normalization) by an iterative subtraction of the average intensities obtained at 0 M condition in each of the cycles of the experiment according to the following formula:

$$SI_{(t)} = X_{(t)} - \sum_{i=1}^{n} \frac{C_{i,(t)}}{n}$$

where X(t) represents the signal intensity of a particular condition at a given time point t, $C_{i,(t)}$ represents the signal intensity of the non-template control (0 M) at the same time point t, and n represents the number of replicates performed at the aforementioned time point t. The limit of detection (LOD) at the end of the reaction was calculated as:

$$LOD = 3.3 \frac{S_y}{Slope}$$

where $S_y$ represents the standard error of the predicted y-value for each x in the regression and Slope represents the slope value (a) of the calibration plot y= ax + b. The estimation of $S_y$ was performed with the STEYX function according to the following formula:

$$S_y = \sqrt{\frac{1}{(n-2)}\left[\sum(y-\bar{y})^2 - \frac{[\sum(x-\bar{x})(y-\bar{y})]^2}{\sum(x-\bar{x})^2}\right]}$$

where x and y are the sample means average (known_x's and known_y's), and n is the sample size.

## 1.4. Specificity assays

To test the specificity of the FokI-assisted signal amplification assay, different combinations of DNA or RNA oligonucleotides corresponding to orthologous sequences of SARS-CoV-2, SARS-CoV or MERS coronaviruses were tested against different custom oligonucleotides. Specificity for SARS-CoV-2 was assayed using the Spike oligonucleotide and related DNA/RNA sequences as for in the basic FokI-assisted signal amplification assay (see previous section). In addition, a second custom oligonucleotide, complementary to MERS Spike protein, but not to other human Beta coronaviruses, was designed and labelled with HEX fluorophore and BHQ-1 quencher, respectively, at its 5' and 3' end. Other oligonucleotides, complementary to the wild-type (N501) or the alpha (N501Y) SARS-CoV-2 variants, were also designed and labelled with FAM and HEX fluorophores at their 5' and 3' ends, respectively (Table 3). All reactions were performed in quadruplicate in a final reaction volume of 20 μl and at a constant temperature of 37ºC. The final concentrations of the reaction components were 2 μl of NEB 10× Cutsmart buffer (New England Biolabs, MA, USA), 5 units of FokI RE (New England Biolabs, MA, USA), 50 nM of custom oligonucleotide complementary to either SARS-CoV-2 variants or MERS synthetic regions, and 5 nM of each target DNA, target RNA or mock sequences in each corresponding reaction, with the exception of the Control DNA/RNA, which was assayed at 50 nM (10-fold excess). The different reactions were followed by the recording over time of the fluorescence intensity, as mentioned above, but the excitation and emission wavelengths were configured to detect FAM and VIC channels simultaneously (488 nm excitation, 538 nm emission, and 554 nm excitation, 549 nm emission, respectively). HEX dye has an excitation/emission spectrum similar to VIC dye (535 nm excitation and 556 nm emission). The conditions of the specificity assay, as well as the subsequent normalization steps are identical to those indicated in the previous section. In the case of the multiplexed reactions, a similar set of experiments was performed. However, these reactions simultaneously incorporated 50 nM of SARS-CoV-2 / MERS or SARS-CoV-2 / SARS-CoV-2 variants custom oligonucleotides, labelled with FAM and HEX fluorophores, respectively.

### 1.5. Padlock probe circularization and molecular coupling between FokI-assisted signal amplification and RCA technologies

To enhance the limit of detection of the FokI-assisted signal amplification reaction, molecular coupling with RCA was performed in two subsequent steps. First, padlock probes containing a phosphate modification at their 5' end were hybridized with their corresponding synthetic RNA molecules, which comprised Spike and Orf8a SARS-CoV-2 target regions (Table 3) and the padlock probe circularization step was performed in triplicate in a reaction volume of 8 $\mu$l. The concentrations of the reaction components were 0.8 $\mu$l of NEB 10× SplintR ligase reaction buffer (New England Biolabs, MA, USA, 1× Buffer composition: 50 mM Tris-HCl, 10 mM Magnesium Chloride, 1 mM ATP, 10 mM Dithiothreitol, pH 7.5 at 25$\underline{o}$C), 5 units of SplintR ligase (New England Biolabs, MA, USA), 10 nM of padlock probe oligonucleotides and variable amounts of each target RNA molecule (1 fmol, 100 amol, 10 amol, 1 amol, 0 amol), with the exception of the Control RNA condition, which was always assayed at 1 fmol. This reaction mixture was incubated for 5 min at room temperature, and then 12 $\mu$l of an RCA premix was added to the resulting reaction. The concentrations and amounts of the RCA premix components were 2 $\mu$l of NEB 10X Cutsmart buffer (New England Biolabs, MA, USA), 40 nM of the universal rolling circle primer, 500 $\mu$M dATP, dGTP, dCTP and dTTP (Promega, WI, USA), 0.5 $\mu$l of QualiPhi DNA polymerase (4Basebio, CA, UK), 0.01 units of Pyrophosphatase Inorganic from *E.coli* (New England Biolabs, MA, USA), 100 nM of 5' FAM and 3'BHQ-1 labelled custom oligonucleotides and 5 units of FokI RE (New England Biolabs, MA, USA), yielding a final reaction volume of 20 $\mu$l. Fluorescence intensity in the FAM channel (excitation 488 nm, emission 520 nm) was recorded over 120 cycles (each cycle 1 minute at 37°C) in a StepOnePlus™ Real-Time PCR System (Applied Biosystems, CA, USA), and fluorescence measurements for the different conditions were subsequently obtained and normalized according to the pipeline described in the FokI-assisted signal amplification assay.

### 1.6. Sample collection and detection of SARS-CoV-2 RNA species in human samples

A total of 111 nasopharyngeal swab samples from patients subjected to COVID19 PCR tests were collected at the Hospital Universitario Central de Asturias (HUCA) with the approval of the Research Ethics Committee of the Principality of Asturias (ref 2020.309). RNA was isolated using a MagNA pure 96 System (Roche Diagnostics, BS, Switzerland) following the manufacturer's recommendations. Detection of SARS-CoV-2 was performed via a coupled RCA - FokI-assisted signal amplification assay as described in the previous section with some minor modifications. To enhance the limit of detection of the reaction, 4 $\mu$l of purified RNA was incubated in the presence of both Spike and Orf8a padlock probes in the padlock circularization step, and their corresponding mixture of 5' FAM and 3' BHQ-1 labelled custom oligonucleotides in the simultaneous RCA / FokI-assisted digestion step. Per-sample background normalization was performed by correcting the signal intensities at the different time points against the signal of each corresponding sample at time 0. Standard scores (z-scores) were calculated according to the following formula:

$$Z_i = \frac{X_i - \overline{X}}{S}$$

where $X_i$ represents the normalized fluorescence intensity of a given sample, and $\overline{X}$ and $S$ represent the sample mean and sample standard deviation, respectively, of the normalized fluorescence intensity obtained from 9 validated pre-pandemic negative control cases at a given time point. Samples with z-scores > 2.5 (p-value < 0.01) were considered to be SARS-CoV-2 positive for viral identification purposes. Amplification and detection of the virus using orthogonal diagnostic approaches was performed using an in-house real time (RT)-PCR approach. Viral genomes were amplified using TaqMan Fast Virus 1-step Master Mix (Life Technologies, CA, USA) and the CDC recommended nucleoprotein specific primers and VIC-labelled minor groove binding (MGB) probes indicated in Table 3. Amplifications and data analysis were carried out in a StepOnePlus™ Real-Time PCR System (Applied Biosystems, CA, USA) under the following conditions: retrotranscription at 50$\underline{o}$C for 15 minutes, denaturation at 95$\underline{o}$C for 5 minutes, 40 cycles at 95$\underline{o}$C for 10 seconds followed by 60$\underline{o}$C for 20 seconds.

## 2. Results

### 2.1 Hairpin guide oligonucleotides hijack native FokI activity in the presence of DNA duplexes and DNA: RNA hybrids

The catalytic domain of FokI might recognize and cleave the DNA strand in the context of an RNA: DNA heteroduplex substrate when fused to a given zinc-finger motif (Kim, Y.G., Shi,Y., Berg,J.M. and Chandrasegaran,S. (1997) Site-specific cleavage of DNA-RNA hybrids by zinc finger/FokI cleavage domain fusions. Gene, 203, 43-49). To interrogate the potential of native FokI to cleave DNA duplexes as well as RNA: DNA hybrids, inventors designed short complementary DNA: DNA or DNA: RNA oligonucleotides labelled with either Cy5.5 or IRD800 dyes at their 5' end and containing a single FokI restriction site (Figure 1A and Figure 1B). As expected, FokI cleaved both substrate strands of a DNA duplex and the cleavage occurred with high efficiency and at defined positions within the target sequence (Figure 1A). However, FokI-mediated cleavage of the heteroduplex DNA: RNA substrate did not yield any identifiable product even after long incubation times (Figure 1B). To rule out any potential issues related to the star activity of the FokI RE, inventors generated new unrelated hybridization sequences that lacked FokI restriction site (Figure 2A and Figure 2B). Inventors demonstrated that the digestion of either DNA duplexes or DNA: RNA heteroduplexes by FokI RE was completely eradicated in the absence of a cognate restriction site, indicating that the cleavage reaction mediated by a native FokI is highly specific and depends on the presence of a DNA duplex and not a DNA: RNA hybrid in the recognition site itself.

To check whether the catalytic domain of FokI may still retain some activity in the absence of a suitable recognition site, inventors adopted a different strategy inspired by the structure of the hairpin guide oligonucleotides proposed by *Weizmann et al., 2006.* These structures retain a double stranded FokI recognition site in one of the oligonucleotides and have an overhang that can hybridize in a sequence specific manner with additional complementary sequences. As FokI cleavage occurs 9-13 nt away from the recognition site, a virtual digestion of the cognate duplex may be achieved in the presence of such structures. Inventors demonstrated that FokI mediated digestion of a DNA duplex using this hairpin guide oligonucleotide yielded the desired restriction product in one of the strands (Figure 1C), although the efficiency of the reaction was reduced 3- to 4-fold (-30% digested product) as compared with the kinetics of the native FokI endonuclease in the presence of a fully complementary substrate (Figure 1A). Interestingly, they did not observe any digestion product on the complementary strand, indicating that in the context of a DNA duplex the hairpin guide oligonucleotide was able to digest itself in the presence of a complementary sequence but not in the absence of the complementary counterpart (Figure 2C). This result indicates that the RE activity of FokI may target unrelated trans sequences by modulating the hybridization properties of the guiding oligonucleotides. Inventors thus adopted a similar strategy to test the potential of FokI to digest DNA: RNA hybrids. In the presence of a complementary RNA oligonucleotide, the hairpin guide oligonucleotide was able to induce a FokI relaxation, which resulted in the partial digestion of the hairpin guide oligonucleotide (Figure 1D). Despite the low efficiency of this process (20- to 25-fold less efficient compared to native FokI with fully complementary DNA duplex, Figure 1A), the cleavage product was consistently and reproducibly detected. These results indicate that the catalytic activity of this RE can be uncoupled from its DNA binding domain for the development of novel molecular strategies for the detection of nucleic acid targets.

### 2.2. FokI-assisted digestion enhances the detection of DNA: RNA heteroduplexes

The use of hairpin guide oligonucleotides can hijack the binding activity of native FokI, and FokI induced relaxation might be used as a desirable system for the virtual digestion of any complementary sequence of interest. In addition, the asymmetric digestion of the substrates observed in the presence of these oligonucleotides rendered a potential scenario for fluorescent signal amplification purposes. FokI-assisted digestion could enhance the detection of nucleic acids, either DNA or RNA, when these hairpin guide, custom oligonucleotides are conjugated to a fluorescent dye-quencher pair. Figure 3 shows the details of the FokI-assisted reaction using a combination of a fluorescent dye and quencher molecules at the 5' and 3'ends of the custom structure [A]. In a conventional hybridization assay, the absence of the target complementary molecule causes a Förster resonance energy transfer (FRET), which inhibits the fluorescence of the molecular beacon due to the proximity of the fluorescent dye-quencher pair. However, in the presence of the target sequence [B], one molecule of nucleic acid substrate can hybridize with one molecule of the custom oligonucleotide generating a duplex/heteroduplex substrate [AB] and releasing the fluorescent signal of the reporter molecule. The implementation of the FokI-assisted digestion system, by means of the combination of custom structures and native FokI RE, may enhance the detection of nucleic acids as the cleavage reaction and the consequential release of the fluorescent signal is dependent on the hybridization between these oligonucleotides and the target sequence [AB*]. The asymmetric cleavage performed by FokI in this context might allow for the rapid release of the fluorescent portion of the probe [C] and the potential recirculation of the target sequence [B] for further rounds of digestion, thus improving on the 1:1 relationship observed with conventional hybridization assays.

To achieve greater enhancement of the signal amplification properties of the system, inventors also implemented an additional strategy for signal amplification mediated by a DNA machine, as previously postulated by *Weizman et al., 2006.* In this context, the byproducts from the FokI-assisted reaction [D*] can still be used for further rounds of digestion in the presence of universal hairpin beacon oligonucleotides [E]. These sequences are labelled with fluorescent dyes and quencher molecules at their 5' and 3' ends, respectively and contain an internal FokI recognition site and a 5' end overhang that can hybridize with the resulting product of the FokI-assisted reaction [D*]. The resulting substrate [DE*] can be further digested by circulating FokI RE units, releasing the fluorescent part of the universal hairpin beacon oligonucleotide [F] and generating two different byproducts [G and H]. As one of the byproducts [H] still retains a FokI recognition site and a 5' overhang which can hybridize with remaining universal hairpin beacon oligonucleotides [E], the new set of actionable substrates [HE*] can be further digested by FokI RE, generating the aforementioned G and H byproducts and creating a self-sustaining loop for signal amplification purposes.

In order to test the efficacy of the proposed signal amplification methods, and to assay whether this system might be used as an alternative system for the detection of real-world pathogens, inventors focused on the study of the recently isolated human Beta coronavirus SARS-CoV-2. First, they scanned the complete SARS-CoV-2 genome for the presence of 6-mer consensus sequences in order to design synthetic RNA and DNA molecules and SARS-CoV-2 complementary custom oligonucleotides able to hybridize with the overhang structure of a common universal hairpin beacon oligonucleotide. Second, they performed a filtering approach to select those regions with maximized sequence divergence between different human coronaviruses. And third, among the resulting candidates, they selected those sequences that were characterized for the presence of 6 bp in the stem structure of the custom oligonucleotide in order to avoid self-cleavage by FokI prior to the hybridization step. This strategy led to the design of 3 custom oligonucleotides, labelled with 6-FAM and BHQ1 at their 5' and 3' ends, respectively, and of their complementary synthetic DNA and RNA substrates, each corresponding to the coding regions of the Nsp4, Spike and Orf8 proteins of SARS-CoV-2 (Figure 4A and Figure 5A, 5B and 5C).

Inventors then examined the performance of the proposed signal amplification strategies in the presence of different DNA or RNA target concentrations. The real-time detection of the fluorescence signal allowed them to analyse the kinetics of the reaction in the context of a conventional hybridization assay, a basic FokI-assisted signal amplification assay or an extended FokI-assisted signal amplification assay in the presence of a self-sustained DNA machine powered by a universal hairpin beacon (UB) oligonucleotide (Figure 4B). They observed that the extent of the signal amplification was sequence dependent, and that the custom oligonucleotide designed against the Spike region resulted in the greatest improvement in fluorescent signal amplification when coupled with FokI-assisted digestion, as compared to conventional hybridization assays (Fig. 6). In addition, the simultaneous combination of multiple custom oligonucleotides and target sequences (Spike and Orf8a) in the same reaction mixture resulted in a further enhancement of the detection signal (Figure 4B) as compared to the aforementioned singleplexed reactions (Figure 6). In fact, inventors observed that regardless of the target context, the presence of the universal hairpin beacon oligonucleotide slightly improved the detection of the fluorescent signal, particularly in the case of RNA molecules, although it was far from being the theoretically expected enhancement generated by the activation of multiple feedback loops. The improved detection of RNA molecules was enhanced by both the basic and the extended FokI-assisted signal amplification assay. Indeed, for the case of the combined Spike and Orf8 regions, the limit of detection was improved more than 3.5-fold in the case of DNA and 3-fold for RNA targets (Figure 4C, LOD conventional assay DNA / RNA = 0.6 nM and 0.91 nM, LOD basic FokI-assisted digestion DNA / RNA = 0.16 nM and 0.27 nM, LOD extended FokI-assisted digestion DNA / RNA = 0.13 nM and 0.20 nM respectively).

## 2.3. Specificity and multiplex capabilities of the FokI-assisted signal amplification assay

To test whether the proposed FokI-assisted digestion of DNA: RNA hybrids show high specificity in distinguishing SARS-CoV-2 targets from divergent orthologous sequences, inventors performed a similar set of experiments in the presence of complex DNA or RNA mixtures from various human Beta coronaviruses. As proof of concept, they focused on the custom oligonucleotide designed against the Spike region of SARS-CoV-2 as this construct achieved the best performance in terms of signal amplification in the context of basic FokI-assisted digestion assays. They also designed an additional custom oligonucleotide specific to the Spike region of MERS (Figure 5D). The latter construct was labelled with HEX fluorophore and BHQ1 quencher at its 5' and 3' end, respectively, to avoid signal interference and to facilitate the detection of the different target molecules for a given fluorescence channel. These custom structures displayed perfect sequence homology with SARS-CoV-2 and MERS but contained a number of mismatches compared to other orthologous sequences of the SARS-CoV and MERS human coronaviruses (Figure 7A and 8A, Figure 5A, 5B, 5C and 5D) which were included for control purposes. Regardless of the nucleic acid complexity of the sample mixture, the enhanced detection of SARS-CoV-2 sequence was only achieved in the presence of the corresponding DNA or RNA target molecule and the custom oligonucleotide in the FAM channel (Figure 7B and 8B, upper left panel). However, the detection of other SARS-CoV-2 related sequences, corresponding to the orthologous sequences of SARS-CoV, MERS (SARS-CoV C1, SARS-CoV C2, MERS C) or unrelated control substrates, was eradicated under these experimental conditions. In addition, no signal was detected in the HEX channel (Figure 7B and 8B, lower left panel), indicating that the reporter custom oligonucleotide efficiently exerts its fluorescence in the corresponding emission spectra. Conversely, detection of MERS by its corresponding custom oligonucleotide was equally well achieved regardless of the complexity of the DNA or RNA mixture, and the detection of MERS and no other related orthologous sequences (SARS-CoV C1, SARS-CoV C2, SARS-CoV-2 C) was obtained in the range of the HEX channel (Figure 7B and 8B, bottom right panel). These results were observed in the presence of complementary DNA or RNA substrates (Figure 7B or 8B respectively), and they indicate that the signal amplification mediated by FokI cleavage is highly specific regardless of the complexity of the samples.

A combination of custom structures and their corresponding nucleic acid substrates could be used to detect multiple targets simultaneously. Thus, inventors combined in the same reaction the aforementioned custom oligonucleotides that target SARS-CoV-2 or MERS, which were labelled with 6-FAM or HEX, respectively, at their 5' end, and a mixture of DNA or RNA substrates including either SARS-CoV-2, MERS, or a combination of both synthetic targets (Figure 9A and 10A, respectively, for DNA duplex or DNA: RNA hybrid detection). As expected, the detection of each single DNA or RNA condition of SARS-CoV-2 or MERS (black lines) was achieved only in the presence of corresponding fluorescent channel (FAM or HEX, respectively), despite the reaction mixture containing both reporter custom oligonucleotides (Figure 9A, 10A). The same was also observed in the context of complex nucleic acid mixtures that included the individual DNA or RNA condition of SARS-CoV-2 or MERS in the presence of other related orthologous control sequences, indicating that this system can discriminate their complementary nucleic acids regardless of the complexity of the sample. Interestingly, samples containing both SARS-CoV-2 and MERS sequences were successfully detected in both fluorescent channels, this dual detection being similarly achieved in the presence of either DNA or RNA substrates (Figure 9A and 10A respectively), thereby validating the multiplexing potential of the FokI-assisted signal amplification assay.

**2.4. FokI-assisted signal amplification is able to discriminate between currently known SARS-CoV-2 variants**

The FokI-driven endonuclease reaction was able to discriminate between similar and related human coronaviruses. However, the emergence of novel SARS-CoV-2 variants, which often differ by only a single nucleotide, represents a more challenging scenario. Thus, to test whether the FokI-assisted signal amplification assay was specific enough to discriminate between recently described SARS-CoV-2 variants, inventors designed novel reporter custom structures which were specific to either the wild type or the B.1.1.7 strains (labelled with FAM or HEX at their 5' end, respectively) and their corresponding synthetic DNA target sequences in the vicinity of the Spike N501Y mutation (Figure 11A, Table 3). When these reactions were performed with their specific, single custom structures, signal amplification was achieved with both wild type and B.1.1.7 target molecules, but not with control sequences, in their corresponding FAM and HEX fluorescent channels (Figure 11B, left and middle panels), although the signal intensity of their fully complementary cognate molecules always outperformed that detected in the context of single nucleotide mismatches. Inventors therefore tested whether the multiplex addition of both custom oligonucleotides in the same reaction could compete with and enhance the detection of a given target molecule. As well as the fact that the simultaneous presence of both fluorescent-labelled custom structures still resulted in both wild type and mutant target molecules being detected, they also observed an improvement in the discrimination of the target sequences in their corresponding channels (Figure 11B, right panel) as compared to the singleplex scenario (Figure 11B, left and middle panels). Interestingly, the multiplex combination of FAM and HEX signal trajectories in a given sample, calculated by means of a principal component analysis, displayed a clear, time-dependent divergence between the two sequence variants (Figure 11C), indicating that the combination of multiple reporter custom structures specific to the different SARS-CoV-2 variants in a single mixture can facilitate the discrimination of SARS-CoV-2 strains with minimal sequence divergence.

**2.5. Improved detection of target molecules in the context of coupled FokI-assisted signal amplification and isothermal amplification of nucleic acids**

Inventors set up a molecular coupling using the RCA technique. This method relies on the isothermal amplification of circular DNA targets mediated by the high processivity and strand displacement activity of the Phi29 DNA polymerase. Figure 12A shows the details of the RCA reaction, which involves the use of padlock probes [A] and a cognate nucleic acid target [B]. The hybridization of a target molecule with a padlock probe reorganizes the configuration of the padlock probe, which adopts a circular structure [AB]. The catalytic activity of a DNA ligase enzyme facilitates the full circularization of the padlock probe by ligating its splinted 5' and 3' ends [C]. The resulting single stranded DNA template can be used for indefinite rounds of amplification in the presence of a complementary RCA primer [D], dNTPs and the DNA polymerase Phi29, thus increasing the number of target sequences in the reaction mixture [E]. Within this context, inventors hypothesized that the simultaneous molecular coupling of the FokI-assisted signal amplification assay might improve on the detection results obtained in classical RCA approaches. Target molecules [B, E] can hybridize with the custom designed structures [F] and generate a duplex/heteroduplex substrate [BF, EF] that can release the fluorescent signal of the reporter molecule. The asymmetric cleavage reaction mediated by FokI can enhance the excision of the fluorescent portion of the probe [G] and the potential reuse of the target sequence [B, E] for further rounds of digestion, thus improving the detection signal of reaction.

To test the efficiency of the padlock probe circularization approach for the detection of SARS-CoV-2 sequences, inventors designed custom DNA padlock oligonucleotides that complemented the Spike and Orf8a regions of SARS-CoV-2 in the vicinity of the sequences designed for the custom oligonucleotides (Figure 12A). Incubation of these padlock probes with their RNA targets and the SplintR DNA ligase led to successful circularization in the presence of their corresponding target molecules, but not with unrelated RNA sequences (Figure 12B). The specificity of the Spike padlock probes was further confirmed using RNA sequences corresponding to the SARS-CoV-2 Spike region or orthologous RNAs from closely related coronaviruses (SARS-CoV, MERS), where full template circularization was observed only in the presence of SARS-CoV-2 RNA, and not when only other divergent coronavirus species were present (Figure 13B).

The subsequent molecular coupling of FokI-assisted digestion and the RCA reaction at short incubation times (< 60 minutes) enhanced the detection limit of the basic FokI-assisted signal amplification assay by 2 to 4 orders of magnitude depending on the target RNA sequence used (Orf8a or Spike), with the custom oligonucleotide designed against the Spike region being, once again, the construct that displayed the greatest improvement in fluorescent signal amplification (Figure 12C). It is worth noting that the presence of the FokI endonuclease in the reaction mixture greatly outperformed the response of the fluorescence signal alone and reduced the detection time as compared with a classical RCA approach. In fact, the absence of the endonuclease did not induce tangible changes in FAM fluorescence between conditions, even after 120 minutes of incubation time (Figure 13C). The simultaneous combination of both custom oligonucleotides and target sequences (Spike and Orf8a) resulted in an additive enhancement of the detection signal (Figure 12C, right panel) as compared to that of singleplex reactions. Under these conditions, inventors achieved a theoretical limit of detection of SARS-CoV-2 RNA of $2.8 \times 10^5$ molecules per millilitre (Figure 13D), a level resembling the conditions observed in the clinical practice.

## 2.6. Detection of SARS-CoV-2 RNA in human samples

Given the improvements in signal amplification achieved with approaches combining RCA and FokI-assisted amplification, inventors further interrogated whether this molecular coupling could be used as a diagnostic method for the detection of SARS-CoV-2 particles in RNA from human samples. They initially tested whether their original custom structures were able to identify the presence of viral sequences in the context of the multiple SARS-CoV-2 variants isolated from nasopharyngeal swabs, including the WT, B.1.1.7, B.1.351, P.1 and B.1.617.2 strains, as well as other pre-pandemic known human coronaviruses (Figure 14A). They achieved a successful detection of SARS-CoV-2 RNA irrespective of the type of variant interrogated, and such signal amplification was not detected in pre-pandemic controls, indicating that the designed structures were highly specific for SARS-CoV-2 and resilient to recently identifies mutations. Inventors further extended this analysis to a cohort of 46 control samples and 65 diagnosed COVID19 cases that included different variants of interest (Figure 14B). The results indicated a significant anti-correlation between the signal intensity of positive SARS-CoV-2 cases obtained in these FokI-assisted signal amplification assays and sample cycle thresholds (Cts) obtained by RT-PCR approaches (Figure 14C), indicating good agreement between the two diagnostic technologies. Significant differences in the signal intensity of SARS-CoV-2 positive and negative cases were observed at reaction times of 45 minutes (Figure 14D), and the accuracy, sensitivity and specificity of the FokI-assisted signal amplification reaction as compared to the gold standard RT-PCR approach were, respectively, 0.84, 0.77 and 0.93 (Figure 14E). These results demonstrate the feasibility of using the proposed method for the detection of viral targets, and that it would provide a cost-effective, time-saving and highly-specific diagnostic strategy for the surveillance of SARS-CoV-2 infection rates in the human population.

## Claims

1.   An *in vitro* method for detecting the presence of at least one single-stranded nucleic acid, preferably a single-stranded DNA (ssDNA) virus or a single-stranded RNA (ssRNA) virus, more preferably a ssRNA virus, in an isolated sample from a subject, wherein said method comprises:

   (I) incubating the sample in the presence of a FokI endonuclease and at least one molecular beacon, wherein said beacon comprises:

      a) a double-stranded DNA fragment comprising a FokI recognition site;

b) an oligonucleotide complementary to a nucleic acid region of the single-stranded nucleic acid;
c) a fluorophore at one of the ends, preferably at the 5' end; and
d) a fluorescence quencher molecule at the end opposite to the end of (c), preferably at the 3' end;

(II) determine whether said single-stranded nucleic acid is present in the sample by determining whether a fluorescent signal of said fluorophore has become detectable.

2. Method according to claim 1, wherein the ssRNA virus is one that causes nasal and/or oral infections or has an oral and/or nasal presence in the subject.

3. Method according to any of claims 1 or 2, wherein the ssRNA virus is selected from the list consisting of: *Influenza A virus, Influenza B virus, Influenza C virus, Alphacoronavirus, Betacoronavirus,* SARS-CoV-2, *Rhinoviruses, Parechovirus,* Parainfluenza and *Pneumovirus.*

4. Method according to any of claims 1 to 3, wherein the fluorophore of the molecular beacon is FAM, HEX, TET, JOE, VIC, Cy3, NED, TAMRA, Cy3.5, ROC, Texas Red or Cy5, preferably FAM or HEX.

5. Method according to any of claims 1 to 4, wherein the quencher of the molecular beacon is BHQ1 or BHQ2, preferably BHQ1.

6. Method according to any of claims 1 to 5, wherein the isolated sample from a subject is a biological sample from a mammal, preferably a human.

7. Method according to any of claims 1 to 6, wherein the biological sample is isolated from the oral or respiratory tract of the subject.

8. Method according to any of claims 1 to 7, wherein the method also includes a previous amplification step, preferably an isothermal amplification, more preferably rolling circle amplification (RCA).

9. Method according to claim 8, wherein the step of isothermal amplification comprises incubating the sample in the presence of means that allow this amplification, wherein said means comprise: at least one padlock probe, which contain a phosphate modification at its 5' end, a Phi29 DNA polymerase or a form of the Phi29 DNA polymerase, and a DNA ligase.

10. Method according to any of claims 8 or 9, wherein the step of isothermal amplification is carried out during less than 70 minutes and at a temperature between 30-40ºC, preferably for 60 minutes at 37ºC.

11. Method according to any of claims 1 to 10, wherein the method is for detecting, in the isolated sample, the presence of more than one ssRNA virus or more than one RNA region of the same ssRNA virus, wherein more than one molecular beacon is used each comprising a different oligonucleotide complementary to a different RNA region of the same ssRNA virus or a different oligonucleotide complementary to a different ssRNA virus.

12. Method according to any of claims 1 to 11, wherein the ssRNA virus is SARS-CoV-2, preferably wild type and/or any of its variants, more preferably the strains B.1.1.7, B.1.351, P.1 or B.1.617.2; and the RNA regions of SARS-CoV-2 are Spike and/or Orf8 proteins.

13. Method according to claim 11, wherein different molecular beacons are labelled with different fluorophores, preferably FAM, HEX, TET, JOE, VIC, Cy3, NED, TAMRA, Cy3.5, ROC, Texas Red or Cy5, more preferably FAM or HEX.

14. A kit comprising means for detecting *in vitro* the presence of at least one single-stranded nucleic acid, preferably a ssDNA or a ssRNA virus, more preferably a ssRNA virus, in an isolated sample from a subject, wherein said means comprise:

i) a FokI endonuclease; and
ii) at least one molecular beacon, wherein said beacon comprises:

a) a double-stranded DNA fragment comprising a FokI recognition site;
b) an oligonucleotide complementary to a nucleic acid region of the single-stranded nucleic acid;

c) a fluorophore at one of the ends, preferably at the 5' end; and
d) a fluorescence quencher molecule at the end opposite to the end of (c), preferably at the 3' end.

**15.** Kit according to claim 14, further comprising means for amplifying the nucleic acid target, preferably by rolling circle amplification, wherein said means comprises:

i) at least one padlock probe, said padlock probe being an oligonucleotide having a phosphate modification at their 5';
ii) a DNA ligase; and
iii) a Phi29 DNA polymerase or a form of the Phi29 DNA polymerase.

**16.** Use of the kit according to any of claims 14 or 15, in the method according to any of claims 1 to 13.

## Fig. 1

**A**

**B**

**C**

**D**

# Fig. 2.

**A**

**B**

**C**

## Fig. 3.

## Fig. 4

**A**

## Fig. 4 (continuation)

## Fig. 5.

**A**

SARS-CoV-2 → Spike (O8)

| | | |
|---|---|---|
| GA + + GTAAAACATTTTC + A | Consensus |
| AACAGGAAGAGAATCAGCA | SARS-CoV-2 / NC_045512.2 |
| GAGAGAAAAAAAATTTCTA | SARS-CoV / NC_004718.3 |
| AAGCGTTTGGTTTTTACCA | MERS / KT121573.1 |
| GAACGTAAAATTTTTTCTA | HKU1 / KF686346.1 |
| GAACGTAAGACATTTTCAA | OC43 / AY391777.1 |
| GTTGACACATCACACTTCA | 229E / KU291448.1 |
| GTTAGAACATCTCATTTTT | NL63 / JX504050.1 |

**B**

SARS-CoV-2 → Nsp4 (O12)

| | | |
|---|---|---|
| TTTTGGC + ATGTCTGCT | Consensus |
| CTTTGGACATATCAGCA | SARS-CoV-2 / NC_045512.2 |
| CTTTAGATGTGTCTGCT | SARS-CoV / NC_004718.3 |
| ATTTCCAATTGACTACC | MERS / KT121573.1 |
| TCTTTTCTCTTACTGCT | HKU1 / KF686346.1 |
| TTTTGGCATTGACTGCT | OC43 / AY391777.1 |
| TTGCTGCAATGTCAGGT | 229E / KU291448.1 |
| TTGTGGCTATGTCTGGA | NL63 / JX504050.1 |

**C**

SARS-CoV-2 → Orf8 (O15)

| | | |
|---|---|---|
| + + + A + C + + G + A + A + CAGCA | Consensus |
| AGGAGCTAGAAAATCAGCA | SARS-CoV-2 / NC_045512.2 |
| TCAAACATGCACACC - - - - | SARS-CoV / NC_004718.3 |
| - - - - - - - - - - - - - - - - - - - | MERS / KT121573.1 |
| - - - - - - - - - - - - - - - - - - - | HKU1 / KF686346.1 |
| - - - - - - - - - - - - - - - - - - - | OC43 / AY391777.1 |
| - - - - - - - - - - - - - - - - - - - | 229E / KU291448.1 |
| - - - - - - - - - - - - - - - - - - - | NL63 / JX504050.1 |

**D**

MERS-CoV → Orf5 (O18)

| | | |
|---|---|---|
| + + TCTACAA + CA + TTT + ACT | Consensus |
| AGACTATTACCAGCTGTACT | SARS-CoV-2 / NC_045512.2 |
| AGTTTACTACCAGCTTGAGT | SARS-CoV / NC_004718.3 |
| GTTTGACATGCGTTCTCACT | MERS / KT121573.1 |
| - - - - - - - - - - - - - - - - - - - - | HKU1 / KF686346.1 |
| - - - - - - - - - - - - - - - - - - - - | OC43 / AY391777.1 |
| GCTCTACAAGACATGGGATT | 229E / KU291448.1 |
| TCTCTATAAAAATTTTTCAT | NL63 / JX504050.1 |

## Fig. 6

# Fig. 7.

**A**

**B**

## DNA

**Fig. 8.**

A

B

## Fig. 9.

**A**

**B**

|  | | | | | | | | | |
|---|---|---|---|---|---|---|---|---|---|
| (O9) SARS-CoV-2 | + | + | + | - | (O9) SARS-CoV-2 | - | - | + | - |
| (O19) SARS-CoV C1 | - | + | + | - | (O19) SARS-CoV C1 | - | - | - | - |
| (O20) MERS C | - | + | - | - | (O20) MERS C | - | - | - | - |
| (O21) Control DNA | - | - | - | + | (O21) Control DNA | - | - | - | + |
| (O22) SARS-CoV-2 C | - | - | - | - | (O22) SARS-CoV-2 C | - | + | - | - |
| (O23) SARS-CoV C2 | - | - | - | - | (O23) SARS-CoV C2 | - | + | + | - |
| (O24) MERS | - | - | + | - | (O24) MERS | + | + | + | - |
| FAM Channel | Y | Y | Y | N | FAM Channel | N | N | Y | N |
| HEX Channel | N | N | Y | N | HEX Channel | Y | Y | Y | N |

## Fig. 10.

**A**

**B**

| | | | | | | | | | |
|---|---|---|---|---|---|---|---|---|---|
| (O10) SARS-CoV-2 | + | + | + | - | (O10) SARS-CoV-2 | - | - | + | - |
| (O25) SARS-CoV C1 | - | + | + | - | (O25) SARS-CoV C1 | - | - | - | - |
| (O26) MERS C | - | + | - | - | (O26) MERS C | - | - | - | - |
| (O27) Control RNA | - | - | - | + | (O27) Control RNA | - | - | - | + |
| (O28) SARS-CoV-2 C | - | - | - | - | (O28) SARS-CoV-2 C | - | + | - | - |
| (O29) SARS-CoV C2 | - | - | - | - | (O29) SARS-CoV C2 | - | + | + | - |
| (O30) MERS | - | - | + | - | (O30) MERS | + | + | + | - |
| FAM Channel | Y | Y | Y | N | FAM Channel | N | N | Y | N |
| HEX Channel | N | N | Y | N | HEX Channel | Y | Y | Y | N |

**Fig. 11.**

**A**

**B**

## Fig. 11. (continuation)

C

## Fig. 12.

A

B

## Fig. 12. (continuation)

**C**

## Fig. 13.

**A**

**B**

**Fig. 13. (continuation)**

C

(O38)
SARS-CoV-2 MB + phi29

(O38)
SARS-CoV-2 MB + phi29 + FokI

- RNA 1 fmol (O10)
- RNA 100 amol (O10)
- Control RNA (O27)
- No RNA

D

LOD = 2.46

$y = 0.18x + 2.57$
$R^2 = 0.94$

**Fig. 14.**

A

RCA + FokI    Spike + Orf8

(O38 + O39 + O40)

Variant
- Prepandemic
- WT
- B.1.1.7
- B.1.351
- P1
- B.1.617.2

## Fig. 14. (continuation)

**B**

SARS-CoV-2 positive (n = 65)
- B.1.1.7 (n = 11)
- B.1.351 (n = 3)
- P1 (n = 3)
- B.1.617.2 (n = 47)
- C.37 (n = 1)

SARS-CoV-2 negative (n = 46)
- Negative (n = 37)
- Prepandemic (n = 9)

**C**

**D**

**Fig. 14. (continuation)**

E

## EUROPEAN SEARCH REPORT

**Application Number**

EP 22 38 2308

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | TEJEDOR JUAN R. ET AL: "Enhanced Detection of Viral RNA Species Using FokI-Assisted Digestion of DNA Duplexes and DNA/RNA Hybrids", ANALYTICAL CHEMISTRY, vol. 94, no. 18, 25 April 2022 (2022-04-25), pages 6760-6770, XP055962466, US ISSN: 0003-2700, DOI: 10.1021/acs.analchem.2c00407 Retrieved from the Internet: URL:https://pubs.acs.org/doi/pdf/10.1021/acs.analchem.2c00407> * figures 1-4 * * abstract * * the whole document * | 1-16 | INV. C12Q1/6818 C12Q1/6844 |
| Y | CN 101 338 343 A (INTEC PRODUCTS INC [CN]) 7 January 2009 (2009-01-07) * claim 1 * * abstract * * the whole document * | 1-13,16 | |
| Y | CN 104 342 502 A (FUJIAN INTERNAT TRAVEL HEALTH CARE CT) 11 February 2015 (2015-02-11) * claim 1 * * abstract * * the whole document * | 1-13,16 | TECHNICAL FIELDS SEARCHED (IPC) C12Q |
| Y | RU 2 756 557 C1 (FED GOSUDARSTVENNOE BYUDZHETNOE UCHREZHDENIE FED TSENTR OKHRANY ZDOROV) 1 October 2021 (2021-10-01) * claim 1 * * abstract * * the whole document * | 1-13,16 | |

-/--

The present search report has been drawn up for all claims

| Place of search | Date of completion of the search | Examiner |
|---|---|---|
| Munich | 20 September 2022 | Helliot, Bertrand |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**page 1 of 3**

Europäisches
Patentamt

European
Patent Office

Office européen
des brevets

**EUROPEAN SEARCH REPORT**

Application Number

EP 22 38 2308

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2005/202416 A1 (HORNER JAMES [US] ET AL) 15 September 2005 (2005-09-15)<br>* claims 17,24 *<br>* abstract *<br>* the whole document * | 1-13,16 | |
| Y | CN 111 485 037 A (SHANDONG VOCATIONAL ANIMAL SCIENCE & VETERINARY COLLEGE) 4 August 2020 (2020-08-04)<br>* claim 1 *<br>* abstract *<br>* the whole document * | 1-13,16 | |
| Y | WO 2014/036972 A1 (SNOVA BIOTECHNOLOGIES CO LTD [CN]) 13 March 2014 (2014-03-13)<br>* claim 9 *<br>* abstract *<br>* the whole document * | 1-13,16 | |
| Y | DEIMAN B ET AL: "Efficient amplification with NASBA^(R) of hepatitis B virus, herpes simplex virus and methicillin resistant Staphylococcus aureus DNA", JOURNAL OF VIROLOGICAL METHODS, ELSEVIER BV, NL, vol. 151, no. 2, 1 August 2008 (2008-08-01), pages 283-293, XP022832562, ISSN: 0166-0934, DOI: 10.1016/J.JVIROMET.2008.04.009 [retrieved on 2008-06-02]<br>* figure 1 *<br>* abstract *<br>* the whole document * | 1-13,16 | TECHNICAL FIELDS SEARCHED (IPC) |

-/--

The present search report has been drawn up for all claims

| Place of search | Date of completion of the search | Examiner |
|---|---|---|
| Munich | 20 September 2022 | Helliot, Bertrand |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches
Patentamt

European
Patent Office

Office européen
des brevets

# EUROPEAN SEARCH REPORT

**Application Number**

**EP 22 38 2308**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ZHANG HONGQUAN ET AL: "DNA-Mediated Homogeneous Binding Assays for Nucleic Acids and Proteins", CHEMICAL REVIEWS, vol. 113, no. 4, 11 December 2012 (2012-12-11), pages 2812-2841, XP055962475, US ISSN: 0009-2665, DOI: 10.1021/cr300340p | 14,15 | |
| Y | * figure 10 * * abstract * * the whole document * | 1-13,16 | |
| X | WEIZMANN YOSSI ET AL: "An autonomous fueled machine that replicates catalytic nucleic acid templates for the amplified optical analysis of DNA", NATURE PROTOCOLS, vol. 1, no. 2, 27 June 2006 (2006-06-27), pages 554-558, XP055962498, GB ISSN: 1754-2189, DOI: 10.1038/nprot.2006.78 Retrieved from the Internet: URL:http://www.nature.com/articles/nprot.2006.78> | 14,15 | TECHNICAL FIELDS SEARCHED (IPC) |
| Y | * figure 1 * * abstract * * the whole document * | 1-13,16 | |

The present search report has been drawn up for all claims

| Place of search | Date of completion of the search | Examiner |
|---|---|---|
| Munich | 20 September 2022 | Helliot, Bertrand |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 38 2308

This annex lists the patent family members relating to the patent documents cited in the above-mentioned European search report.
The members are as contained in the European Patent Office EDP file on
The European Patent Office is in no way liable for these particulars which are merely given for the purpose of information.

20-09-2022

| Patent document cited in search report | | Publication date | Patent family member(s) | | Publication date |
|---|---|---|---|---|---|
| CN 101338343 | A | 07-01-2009 | NONE | | |
| CN 104342502 | A | 11-02-2015 | NONE | | |
| RU 2756557 | C1 | 01-10-2021 | NONE | | |
| US 2005202416 | A1 | 15-09-2005 | AU | 2003263028 A1 | 10-11-2003 |
| | | | US | 2005202416 A1 | 15-09-2005 |
| | | | WO | 03091270 A1 | 06-11-2003 |
| CN 111485037 | A | 04-08-2020 | NONE | | |
| WO 2014036972 | A1 | 13-03-2014 | CN | 103667252 A | 26-03-2014 |
| | | | WO | 2014036972 A1 | 13-03-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10815539 B

**Non-patent literature cited in the description**

- **KIM,Y.G. ; CHA,J. ; CHANDRASEGARAN,S.** Hybrid restriction enzymes: zinc finger fusions to Fok I cleavage domain. *Proc. Natl. Acad. Sci. U.S.A.,* 1996, vol. 93, 1156-1160
- **TSAI,S.Q. et al.** Dimeric CRISPR RNA-guided Fokl nucleases for highly specific genome editing. *Nat. Biotechnol.,* 2014, vol. 32, 569-576
- **WEIZMANN,Y. ; CHEGLAKOV,Z. ; PAVLOV,V. ; WILLNER,I.** An autonomous fueled machine that replicates catalytic nucleic acid templates for the amplified optical analysis of DNA. *Nat Protoc,* 2006, vol. 1, 554-558
- **DING X. et al.** *Nat Commun.,* 2020
- **KIM JH. et al.** *Biochip J.,* 2019
- **MILLER,J.R et al.** Aggressive assembly of pyrosequencing reads with mates. *Bioinformatics,* 2008, vol. 24, 2818-2824
- **KIM, Y.G. ; SHI,Y. ; BERG,J.M. ; CHANDRASEGARAN,S.** Site-specific cleavage of DNA-RNA hybrids by zinc finger/Fokl cleavage domain fusions. *Gene,* 1997, vol. 203, 43-49